(19) **Europäisches Patentamt European Patent Office Office européen des brevets**

(11) **EP 4 129 283 A1**

(12) **EUROPEAN PATENT APPLICATION**
published in accordance with Art. 153(4) EPC

(43) Date of publication:
08.02.2023 Bulletin 2023/06

(21) Application number: 21780063.0

(22) Date of filing: 30.03.2021

(51) International Patent Classification (IPC):
*A61K 31/375* (1974.07)    *A61K 45/06* (1974.07)
*A61P 35/00* (2000.01)    *A61P 35/02* (2000.01)
*A61P 35/04* (2000.01)

(52) Cooperative Patent Classification (CPC):
A61K 31/375; A61K 45/06; A61P 35/00;
A61P 35/02; A61P 35/04

(86) International application number:
PCT/CN2021/084008

(87) International publication number:
WO 2021/197333 (07.10.2021 Gazette 2021/40)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 31.03.2020 CN 202010245573

(71) Applicant: Natural Medicine Institute
of Zhejiang Yangshengtang Co., Ltd.
Hangzhou, Zhejiang 310024 (CN)

(72) Inventors:
• XU, Nuo
Hangzhou, Zhejiang 310024 (CN)
• LIN, Jian
Beijing 100871 (CN)

(74) Representative: Böhm, Brigitte
Weickmann & Weickmann
Patent- und Rechtsanwälte PartmbB
Postfach 860 820
81635 München (DE)

(54) **PHARMACEUTICAL COMBINATION AND USE THEREOF**

(57) The present application relates to a pharmaceutical combination, comprising: a) a prophylactically and/or therapeutically effective amount of ascorbic acid or a derivative thereof; and b) a prophylactically and/or therapeutically effective amount of a second therapeutic agent selected from the group consisting of: a Notch signaling pathway inhibitor, an HH signaling pathway inhibitor, a Porcupine inhibitor, an RXRα inhibitor, Niclosamide, a CK1a inhibitor, and a Frizzled receptor inhibitor.

EP 4 129 283 A1

**Description**

**TECHNICAL FIELD**

The present invention relates to the field of biomedicine, in particular to a pharmaceutical combination and use thereof.

**BACKGROUND OF THE INVENTION**

Malignant tumors are diseases that are serious health hazard at present. For example, osteosarcoma as a common malignant bone tumor has an annual incidence of about 3/1,000,000 in China. In general, 20% of patients have developed distant metastasis at the time of diagnosis of osteosarcoma. If the osteosarcoma has developed distant metastasis, the five-year disease-free survival rate of patients is less than 20%. Current drugs for treating osteosarcoma comprises high-dose methotrexate, ifosfamide, doxorubicin, cisplatin, etc. Alternatively, two or more drugs are used in combination for chemotherapy to ensure a sufficient dose intensity. However, these chemotherapy regimens would produce strong side effects that may endanger life and health. Colorectal cancer accounts for 10.2% of the total incidence of cancers and 9.2% of deaths, namely, there are approximately 1,850,000 new cases and 880,000 deaths from colorectal cancer every year. Oxaliplatin is the main clinical treatment for colorectal cancer. However, more than 30% of patients with colon cancer are not sensitive to oxaliplatin. Ovarian cancer accounts for about 3.4% of the malignant tumor incidence, and the clinical chemotherapy regimens comprise platinum-based drugs + cyclophosphamide (PC) and taxol + carboplatin (TP). However, these chemotherapy regimens also have serious side effects, for example, hazards to the digestive system and blood system. These all show that chemotherapeutics exhibit different degrees of toxic and side effects while exhibiting an anti-tumor effect. In addition, some tumor patients are not sensitive to chemotherapeutics. Therefore, it is an urgent problem for the medical community around the world to find more effective anti-tumor drugs and methods.

**SUMMARY OF THE INVENTION**

The present application provides a pharmaceutical combination and use or administration method thereof, primarily comprising using a therapeutic agent including a Notch signaling pathway inhibitor, an HH signaling pathway inhibitor, a Porcupine inhibitor, an RXRα inhibitor, Niclosamide, a CK1a inhibitor and/or a Frizzled receptor inhibitor in combination with ascorbic acid or a derivative thereof as a pharmaceutical combination for treating tumors, or administering a therapeutic agent including a Notch signaling pathway inhibitor, an HH signaling pathway inhibitor, an RXRα inhibitor and/or a Wnt signaling pathway inhibitor in combination with ascorbic acid or a derivative thereof for treating tumors, which has at least one of the following technical effects:

1) the pharmaceutical combination can serve as an alternative to traditional chemotherapeutics to avoid the toxic and side effects caused by the traditional chemotherapy;
2) the pharmaceutical combination is used to treat patients who are not sensitive to traditional chemotherapeutics to achieve therapeutic effects;
3) the pharmaceutical combination can be used in combination with traditional chemotherapeutics to decrease the dose of chemotherapeutics, thereby reducing the toxic and side effects of the traditional chemotherapeutics;
4) the pharmaceutical combination produces an unexpected inhibitory effect on tumors, significantly decreases the dose of single-drug administration with equivalent effect, and improves the drug safety;
5) the pharmaceutical combination specifically inhibits the growth of tumor cells, and would not affect other normal cells (e.g., the 293 cells), indicating the drug safety.

The present application provides a pharmaceutical combination, comprising:

a) a prophylactically and/or therapeutically effective amount of ascorbic acid or a derivative thereof; and
b) a prophylactically and/or therapeutically effective amount of a second therapeutic agent selected from the group consisting of: a Notch signaling pathway inhibitor, an HH signaling pathway inhibitor, a Porcupine inhibitor, an RXRα inhibitor, Niclosamide, a CK1a inhibitor, and a Frizzled receptor inhibitor.

In certain embodiments, the RXRα inhibitor comprises berberine or a pharmaceutically acceptable salt thereof.
In certain embodiments, the Notch signaling pathway inhibitor comprises a γ-secretase inhibitor.
In certain embodiments, the γ-secretase inhibitor comprises MK0752, RO4929097 and/or PF-03084014.
In certain embodiments, the HH signaling pathway inhibitor comprises an SMO inhibitor.

In certain embodiments, the SMO inhibitor comprises Vismodegib, Sonidegib and/or Glasdegib.

In certain embodiments, the Porcupine inhibitor comprises LGK974 and/or ETC-159.

In certain embodiments, the CK1a inhibitor comprises Pyrvinium.

In certain embodiments, the Frizzled receptor inhibitor comprises Foxy-5.

In certain embodiments, the inhibitor comprises Fervenulin, 3-Methyltoxoflavin and/or Walrycin B.

In certain embodiments, the derivative of ascorbic acid comprises a pharmaceutically acceptable ascorbate.

In certain embodiments, the pharmaceutically acceptable ascorbate comprises sodium ascorbate.

In certain embodiments, a ratio of the effective amount of the second therapeutic agent to the effective amount of the ascorbic acid or a derivative thereof is about 1:10 to about 1:5000.

In certain embodiments, a mass ratio of the second therapeutic agent to the ascorbic acid or a derivative thereof is about 1:10 to about 1:5000.

In certain embodiments, the second therapeutic agent and the ascorbic acid or a derivative thereof are each present in different containers.

In certain embodiments, the pharmaceutical combination comprises a first preparation and a second preparation, wherein the first preparation comprises the ascorbic acid or a derivative thereof and a pharmaceutically acceptable first carrier, and the second preparation comprises the second therapeutic agent and a pharmaceutically acceptable second carrier.

In certain embodiments, the pharmaceutical combination comprises a pharmaceutical composition, and the pharmaceutical composition comprises the second therapeutic agent and the ascorbic acid or a derivative thereof.

In certain embodiments, the second therapeutic agent has a content of about 5% to about 20% (w/w) in the pharmaceutical composition.

In certain embodiments, the ascorbic acid or a derivative thereof has a content of about 80% to about 95% (w/w) in the pharmaceutical composition.

In another aspect, the present application further provides a kit comprising the pharmaceutical combination as described in the present application.

In another aspect, the present application further provides use of a combination of ascorbic acid or a derivative thereof with a second therapeutic agent in the manufacture of a drug for preventing and/or treating tumors, wherein the second therapeutic agent is selected from the group consisting of: a Notch signaling pathway inhibitor, an HH signaling pathway inhibitor, an RXRα inhibitor, and a Wnt signaling pathway inhibitor.

In certain embodiments, the Wnt signaling pathway inhibitor comprises a Porcupine inhibitor, a Dvl-2 inhibitor, a CK1a inhibitor, and/or a Frizzled receptor inhibitor.

In certain embodiments, the Porcupine inhibitor comprises LGK974 and/or ETC-159.

In certain embodiments, the Dvl-2 inhibitor comprises Niclosamide and/or Sulindac.

In certain embodiments, the CK1a inhibitor comprises Pyrvinium.

In certain embodiments, the Frizzled receptor inhibitor comprises Foxy-5.

In certain embodiments, the RXRα inhibitor comprises berberine or a pharmaceutically acceptable salt thereof.

In certain embodiments, the Notch signaling pathway inhibitor comprises a γ-secretase inhibitor.

In certain embodiments, the γ-secretase inhibitor comprises MK0752, RO4929097 and/or PF-03084014.

In certain embodiments, the HH signaling pathway inhibitor comprises an SMO inhibitor.

In certain embodiments, the SMO inhibitor comprises Vismodegib, Sonidegib and/or Glasdegib.

In certain embodiments, the tumors comprise solid tumors and non-solid tumors.

In certain embodiments, the solid tumors comprise osteosarcoma and/or colon cancer.

In certain embodiments, the non-solid tumors comprise lymphoma.

In certain embodiments, the derivative of ascorbic acid comprises a pharmaceutically acceptable ascorbate.

In certain embodiments, the pharmaceutically acceptable ascorbate comprises sodium ascorbate.

In certain embodiments, the second therapeutic agent and the ascorbic acid or a derivative thereof are formulated to be simultaneously administered to a subject.

In certain embodiments, the second therapeutic agent and the ascorbic acid or a derivative thereof are formulated to be separately administered to a subject.

In certain embodiments, the drug is formulated so that the second therapeutic agent is administered at a dose of about 1 mg/kg to about 100 mg/kg.

In certain embodiments, the drug is formulated so that the ascorbic acid or a derivative thereof is administered at a dose of about 0.05 g/kg to about 2.5 g/kg.

In certain embodiments, the drug is formulated so that the second therapeutic agent and the ascorbic acid or a derivative thereof are administered at a mass ratio of about 1:5 to about 1:5000.

In certain embodiments, the drug is formulated so that the second therapeutic agent and the ascorbic acid or a derivative thereof are administered at a mass ratio of about 1:5 to about 1 :5000.

In another aspect, the present application further provides a method for preventing and/or treating tumors,

comprising administering to a subject in need thereof:

a) ascorbic acid or a derivative thereof; and
b) a second therapeutic agent, wherein the second therapeutic agent is selected from the group consisting of: a Notch signaling pathway inhibitor, an HH signaling pathway inhibitor, an RXRα inhibitor, and a Wnt signaling pathway inhibitor.

In certain embodiments, the Wnt signaling pathway inhibitor comprises a Porcupine inhibitor, a Dvl-2 inhibitor, a CK1a inhibitor, and/or a Frizzled receptor inhibitor.

In certain embodiments, the Porcupine inhibitor comprises LGK974 and/or ETC-159.

In certain embodiments, the Dvl-2 inhibitor comprises Niclosamide and/or Sulindac.

In certain embodiments, the CK1a inhibitor comprises Pyrvinium.

In certain embodiments, the Frizzled receptor inhibitor comprises Foxy-5.

In certain embodiments, the RXRα inhibitor comprises berberine or a pharmaceutically acceptable salt thereof.

In certain embodiments, the Notch signaling pathway inhibitor comprises a γ-secretase inhibitor.

In certain embodiments, the γ-secretase inhibitor comprises MK0752, RO4929097 and/or PF-03084014.

In certain embodiments, the HH signaling pathway inhibitor comprises an SMO inhibitor.

In certain embodiments, the SMO inhibitor comprises Vismodegib, Sonidegib and/or Glasdegib.

In certain embodiments, the tumors comprise solid tumors and non-solid tumors.

In certain embodiments, the solid tumors comprise osteosarcoma and/or colon cancer.

In certain embodiments, the non-solid tumors comprise lymphoma.

In certain embodiments, the derivative of ascorbic acid comprises a pharmaceutically acceptable ascorbate.

In certain embodiments, the pharmaceutically acceptable ascorbate comprises sodium ascorbate.

In certain embodiments, the second therapeutic agent and the ascorbic acid or a derivative thereof are formulated to be simultaneously administered to the subject.

In certain embodiments, the second therapeutic agent and the ascorbic acid or a derivative thereof are formulated to be separately administered to the subject.

In certain embodiments, the drug is formulated so that the second therapeutic agent is administered at a dose of about 1 mg/kg to about 100 mg/kg.

In certain embodiments, the drug is formulated so that the ascorbic acid or a derivative thereof is administered at a dose of about 0.05 g/kg to about 2.5 g/kg.

In certain embodiments, the drug is formulated so that the second therapeutic agent and the ascorbic acid or a derivative thereof are administered at a mass ratio of about 1:5 to about 1:5000.

In certain embodiments, the drug is formulated so that the second therapeutic agent and the ascorbic acid or a derivative thereof are administered at a mass ratio of about 1:5 to about 1:5000.

In another aspect, the present application further provides a method for monitoring a response to a drug in a subject, comprising:

a) administering to the subject ascorbic acid or a derivative thereof and a second therapeutic agent, wherein the second therapeutic agent is selected from the group consisting of: a Notch signaling pathway inhibitor, an HH signaling pathway inhibitor, an RXRα inhibitor, and a Wnt signaling pathway inhibitor; and
b) detecting a change in one or more of the level and/or activity of blood alkaline phosphatase, the level and/or activity of lactic dehydrogenase, the expression level of CADM1 gene, the expression level of CD44 gene, the expression level of livin gene, the expression level of HIF-1α gene, the expression level of ET-1 gene, the expression level of IGF-1R gene, the expression level of STAT3 gene, the expression level of CEA gene, the expression level of CA199 gene, the expression level of CA125 gene, the expression level of AFP gene, the expression level of CA724 gene, and the expression level of β-HCG gene in the subject after the administration of a).

In certain embodiments, the Wnt signaling pathway inhibitor comprises a Porcupine inhibitor, a Dvl-2 inhibitor, a CK1a inhibitor, and/or a Frizzled receptor inhibitor.

In certain embodiments, the Porcupine inhibitor comprises LGK974 and/or ETC-159.

In certain embodiments, the Dvl-2 inhibitor comprises Niclosamide and/or Sulindac.

In certain embodiments, the CK1a inhibitor comprises Pyrvinium.

In certain embodiments, the Frizzled receptor inhibitor comprises Foxy-5.

In certain embodiments, the RXRα inhibitor comprises berberine or a pharmaceutically acceptable salt thereof.

In certain embodiments, the Notch signaling pathway inhibitor comprises a γ-secretase inhibitor.

In certain embodiments, the γ-secretase inhibitor comprises MK0752, RO4929097 and/or PF-03084014.

In certain embodiments, the HH signaling pathway inhibitor comprises an SMO inhibitor.

In certain embodiments, the SMO inhibitor comprises Vismodegib, Sonidegib and/or Glasdegib.

In certain embodiments, the tumors comprise solid tumors and non-solid tumors.

In certain embodiments, the solid tumors comprise osteosarcoma and/or colon cancer.

In certain embodiments, the non-solid tumors comprise lymphoma.

In certain embodiments, the derivative of ascorbic acid comprises a pharmaceutically acceptable ascorbate.

In certain embodiments, the pharmaceutically acceptable ascorbate comprises sodium ascorbate.

In certain embodiments, the second therapeutic agent and the ascorbic acid or a derivative thereof are formulated to be simultaneously administered to the subject.

In certain embodiments, the second therapeutic agent and the ascorbic acid or a derivative thereof are formulated to be separately administered to the subject.

In certain embodiments, the drug is formulated so that the second therapeutic agent is administered at a dose of about 1 mg/kg to about 100 mg/kg.

In certain embodiments, the drug is formulated so that the ascorbic acid or a derivative thereof is administered at a dose of about 0.05 g/kg to about 2.5 g/kg.

In certain embodiments, the drug is formulated so that the second therapeutic agent and the ascorbic acid or a derivative thereof are administered at a mass ratio of about 1:5 to about 1:5000.

In certain embodiments, the drug is formulated so that the second therapeutic agent and the ascorbic acid or a derivative thereof are administered at a mass ratio of about 1:5 to about 1:5000.

Persons skilled in the art can readily recognize other aspects and advantages of the present application form the detailed description below. The following detailed description only shows and describes exemplary embodiments of the present application. As persons skilled in the art will appreciate, the present application enables persons skilled in the art to make modifications to the disclosed embodiments without departing from the spirit and scope of the invention involved in the present application. Correspondingly, the accompany drawings and description in the specification of the present application are only illustrative, rather than restrictive.

## BRIEF DESCRIPTION OF THE DRAWING

The specific features of the invention involved in the present application are shown in the appended claims. By referring to the exemplary embodiments as described in detail below and the accompanying drawings, the features and advantages of the invention involved in the present application can be better understood. The accompany drawings are briefly described as follows:

**FIG. 1** shows an effect of berberine with different concentrations described in the present application on 143B cells and HOS cells.

**FIG. 2** shows an inhibitory effect of berberine in combination with sodium ascorbate described in the present application on 143B cells and HOS cells.

**FIG. 3** shows an effect of Foxy-5 with different concentrations described in the present application on 143B cells and HOS cells.

**FIG. 4** shows an inhibitory effect of Foxy-5 in combination with sodium ascorbate described in the present application on 143B cells and HOS cells.

**FIG. 5** shows an inhibitory effect of Pyrvinium in combination with sodium ascorbate described in the present application on 143B cells and HOS cells.

**FIG. 6** shows an inhibitory effect of Niclosamide in combination with sodium ascorbate described in the present application on 143B cells and HOS cells.

**FIG. 7** shows an inhibitory effect of ETC-159 in combination with sodium ascorbate described in the present application on 143B cells and HOS cells.

**FIG. 8** shows an inhibitory effect of LGK974 in combination with sodium ascorbate described in the present application on 143B cells and HOS cells.

**FIG. 9** shows an effect of Sonidegib with different concentrations described in the present application on 143B cells and HOS cells.

**FIG. 10** shows an inhibitory effect of Sonidegib in combination with sodium ascorbate described in the present application on 143B cells and HOS cells.

**FIG. 11** shows an inhibitory effect of Vismodegib in combination with sodium ascorbate described in the present application on 143B cells and HOS cells.

**FIG. 12** shows an effect of PF-03084014 with different concentrations described in the present application on 143B cells and HOS cells.

**FIG. 13** shows an inhibitory effect of PF-03084014 in combination with sodium ascorbate described in the present application on 143B cells and HOS cells.

FIG. 14 shows an inhibitory effect of RO4929097 in combination with sodium ascorbate described in the present application on 143B cells and HOS cells.

FIG. 15 shows an inhibitory effect of MK0752 in combination with sodium ascorbate described in the present application on 143B cells and HOS cells.

FIG. 16 shows an effect of Sulindac with different concentrations described in the present application on 143B cells and HOS cells.

FIG. 17 shows an inhibitory effect of Sulindac in combination with sodium ascorbate described in the present application on 143B cells and HOS cells.

FIG. 18 shows an inhibitory effect of Niclosamide in combination with sodium ascorbate described in the present application on MC38 cells.

FIG. 19 shows an inhibitory effect of Niclosamide with different concentrations in combination with sodium ascorbate described in the present application on DB cells.

## DETAILED DESCRIPTION OF THE INVENTION

Hereinafter the embodiments of the invention of the application are described by specific examples. Those skilled in the art can easily understand other advantages and effects of the invention as described in the present application from the disclosure in the description.

## Definitions of Terms

In the present application, the term "Notch signaling pathway" generally refers to a cellular signaling pathway mediated by a Notch receptor. Notch signaling pathway is a highly conserved cellular signal transduction system present in most multicellular organisms. The Notch signaling pathway is mainly composed of Notch receptors, Notch ligands (DSL proteins), CSL (a generic term of CBF-1, Su(H), and Lag) DNA binding proteins, regulatory molecules of Notch, and other effectors. CBF-1, Su(H), and Lag1 are different name of the protein in mammals, *Drosophila,* and *Nematode.* In 1917, Morgan and colleagues discovered the Notch gene in mutant Drosophila flies, which was named because the partial loss of function of the gene would cause a notch (Notch) in the edge of the fly's wings. Mammals have 4 Notch receptors (Notch1, Notch2, Notch3, and Notch4) and 5 Notch ligands (Delta-like 1, Delta-like 3, Delta-like 4, Jagged1 and Jagged2). Studies have shown that the Notch signaling can be generated by an interaction of a Notch ligand with a receptor in neighboring cells. A Notch protein that has undergone tertiary cleavage (e.g., cleavage mediated by a $\gamma$-secretase) is released from the intracellular domain (NICD) into the cytoplasm, and enters the cell nucleus to combine with a transcription factor CSL to form a NICD/CSL transcription activation complex, thereby activating the target gene of a basic-helix-loop-helix (bHLH) transcription repression factor family such as HES, HEY, HERP, etc., and playing a biological role.

In the present application, the term "functional variant" generally means that the functional variant can comprise molecules that are subject to amino acid modification (e.g., group substitution or the like) in the original protein, or insertion, substitution, and/or deletion of one or more amino acids in the original protein sequence while reserving the functional of the original sequence. For example, the functional variant can have better biological activity (function) than that of the original sequence. For example, the reservation need not be a full reservation. For example, the functional variant can substantially reserve the function of the original sequence, e.g., reserving at least 50%, 60%, 65%, 70%, 75%, 80%, 85%, 90%, 95%, 96%, 97%, 98%, or 99% of the function of the original sequence.

In the present application, "$\gamma$-secretase" generally refers to an intramembrane proteolytic enzyme composed of four subunits that can cleave a transmembrane protein at residues within the transmembrane domain. A $\gamma$-secretase complex is commonly composed of four separate protein subunits of presenilin-1, nicastrin, APH-1, and presenilin enhancer 2 or functional variants thereof, wherein the Nicastrin mainly plays role of maintaining the stability of the complex and regulating the intracellular protein transport; the PEN-2 is associated with the complex by binding to the transmembrane domain of presenilin, and helps to stabilize the complex after the presenilin undergoes a proteolysis to produce activated N- and C-terminal fragments, among other possible roles; and the APH-1 is required for proteolytic activity, that binds to the complex via a conserved $\alpha$-helix interaction motif, and helps to initiate the assembly of immature components. The $\gamma$-secretase is involved in the processing of $\beta$-amyloid precursor (APP) and several other type I membrane proteins (e.g., Notch, ErbB4, E-cadherin, N-cadherin, ephrin-B2 and/or CD44). Presenilin as a catalytic subunit is an aspartyl protease.

In the present application, the term "HH signaling pathway" generally refers to a cell signaling pathway mediated by Hedgehog (Hh) signal molecule. In many developmental processes of vertebrates and invertebrates, the Hedgehog signaling pathway controls cell proliferation and differentiation. When this signaling pathway is abnormally activated, it may lead to the occurrence and development of tumors. Hedgehog signal molecule is a local protein ligand secreted by signaling cells. There are three Hedgehog homologous genes in mammals: Sonic Hedgehog (SHH), Indian Hedgehog (IHH), and Desert Hedgehog(DHH), which encode Shh, Ihh, and Dhh proteins, respectively. The Hh protein family members are all composed of two domains: an amino terminal domain (Hh-N) and a carboxyl terminal domain (Hh-C), wherein the Hh-N has a signaling activity of Hh protein, and the Hh-C has its own proteolytic enzyme activity and cholesterol transferase function. The Hh signaling is controlled by two receptors, Patched (Ptc) and Smoothened (Smo), on the membrane of the target cell. The receptor Ptc is encoded by the tumor suppressor gene Patched and composed of a single peptide chain with 12 transmembrane regions, which can directly bind to the ligand and negatively regulates the Hh signaling; and Smo is a receptor required by the Hedgehog (Hh) signaling.

In the present application, the term "SMO" generally refers to a receptor required by the Hedgehog (Hh) signaling in the HH signaling pathway or a functional variant thereof. The receptor Smo is encoded by the proto-oncogene Smoothened, homologous to the G protein-coupled receptor, and composed of a single peptide chain with 7 transmembrane regions, wherein the N-terminus is located outside the cell, the C-terminus is located inside the cell, the amino acid sequences of the transmembrane regions are highly conserved, and the serine and threonine residues at the C-terminus are phosphorylation sites which bind to phosphate group when catalyzed by a protein kinase. Only when maintained at full length, a member of this protein family can function as a transcription promoter to initiate the transcription of downstream target gene. When the carboxyl terminus is hydrolyzed by a proteasome, a transcription suppressor is formed to suppress the transcription of downstream target gene. It has been reported that in the absence of the Hh or Ptc, the activation of Smo may lead to the activation of the Hh target gene; and when the gene Smo is mutated, it may exhibit the same characterizations as in the mutation of the Hh gene.

In the present application, the term "Wnt signaling pathway" generally refers to a cell signaling pathway mediated by the Wnt protein. At present, three Wnt signaling pathways have been identified: a canonical Wnt pathway, a noncanonical Wnt/planar cell polarity pathway (PCP), and a noncanonical Wnt/calcium pathway. The three Wnt signaling pathways are all activated by the binding of the Wnt protein ligand to a receptor thereof, Frizzled receptor. It has been reported that the canonical Wnt signaling pathway can participated in the regulation of gene expression, the noncanonical planar cell polarity pathway can regulate the cytoskeleton to control the cell shape, and the noncanonical Wnt/calcium pathway can regulate the concentration of calcium in the cell. The Wnt signaling pathway is commonly used for the communication between adjacent cells (paracrine) or the communication within the cell itself (autocrine). The Wnt signaling pathway is highly conserved in animals.

In the present application, the term "Dvl-2" generally refers to a member of the dishevelled (dsh) protein family or a functional variant thereof. In humans, the Dvl-2 protein is encoded by the DVL2 gene. The gene encodes a 90kD protein that is subject to post-translational phosphorylation to form a 95kD cytoplasmic protein, which can play a role in signal transduction pathways mediated by a plurality of Wnt proteins. The vertebrate dsh proteins have about 40% amino acid sequence similarity with the *Drosophila* dsh.

In the present application, the term "pharmaceutical combination" generally refers to a combination comprising at least two active ingredients/therapeutic agents. In some embodiments, various active ingredients/therapeutic agents can each be prepared into separated preparations (solid, liquid, gel, etc.); in some embodiments, various active ingredients/therapeutic agents can each be present in different containers, and can be simultaneously or respectively formulated with a suitable carrier to a desired preparation as required; in some embodiments, various active ingredients/therapeutic agents can be derived from different sources (e.g., prepared, produced, or sold by different manufacturers); and in some embodiments, various active ingredients/therapeutic agents can be mixed to form a pharmaceutical composition.

In the present application, the term "CK1a", also known as "casein kinase 1$\alpha$", generally refers to a member of the casein kinase 1 family (EC 2.7.11.1) or a functional variant thereof, which belongs to a serine/threonine kinase (in some cases, the tyrosine residues are also phosphorylated). It is known that the casein kinase 1 has 7 isoforms in mammals including $\alpha$, $\beta 1$, $\gamma 1$, $\gamma 2$, $\gamma 3$, $\delta$ and $\epsilon$ isoforms. It is known that these isoforms activate, deactivate, stabilize, or unstabilize the function of the protein to participate in the regulation of various different functions of an organism by phosphorylating various different substrate proteins. The encoding gene for casein kinase 1$\alpha$ can be found in HGNC: 2451.

In the present application, the term "Frizzled receptor" generally refers to a G protein-coupled receptor family or a functional variant thereof that can serve as the receptor in the Wnt signaling pathway and other signaling pathways. In general, when activated, it can cause the activation of downstream proteins (e.g., Dishevelled) in the intracellular signaling pathway. The Frizzled receptor protein generally comprises a cysteine-rich domain that is commonly found in a protein such as receptor casein kinases. In humans, the Frizzled receptor can comprise eight members, that is, Frizzled receptors 1-8.

In the present application, the term "Porcupine" generally refers to a protein capable of participating in secretion or endoplasmic reticulum transport in humans or a functional variant thereof, which is involved in the Wnt signaling pathway conduction. In *Caenorhabditis elegans*, the homologue thereof, mom-1, has a similar function in promoting the secretion of the Wnt protein Mom-2. The Porcupine has some homology with the o-acyl transferase family, and may be involved in the modification of Wnt proteins. The encoding gene thereof can be found in HGNC: 17652.

In the present application, the term "RXR$\alpha$", also known as NR2B1 (nuclear receptor subfamily 2, group B, member 1), generally refers to a nuclear receptor or a functional variant thereof. In humans, it is encoded by the RXRA gene, and the encoding sequence can be found in MGI: 98214.

In the present application, the term "pharmaceutical composition" generally refers to a mixture comprising at least two active ingredients that are administered to a subject to treat a particular disease or disorder affecting the subject. It allows the active ingredients to be in an effective form and does not comprise additional ingredients which are unacceptably toxic to the subject to which the composition is to be administered. The composition can be sterile, and can comprise a pharmaceutically acceptable carrier.

In the present application, the term "inhibitor" generally refers to a compound/substance or a composition capable of completely or partially blocking or reducing the physiological functions of one or more particular biomolecules (e.g., proteins, polypeptides, lipopolysaccharides, glycoproteins, ribonucleoprotein complexes, or the like). The reduction of the physiological functions of one or more particular proteins can comprise the reduction of the activity of the protein itself (e.g., the ability of bonding to other molecules, etc.) or the reduction of the amount of the protein itself. In certain embodiments, the inhibitor can be present as different crystals, amorphous substance, pharmaceutically acceptable salts, hydrates, and solvates. In certain embodiments, the inhibitor can block the activation of cell signaling pathways.

In the present application, the term "ascorbic acid derivative" generally refers to a compound that releases ascorbic acid (vitamin C) *in vivo* or *in vitro* and solvates, hydrates, and salts thereof. This term further comprises an ascorbic acid analog in which one or more hydroxyl groups in the ascorbic acid are replaced with another moiety, and wherein the ascorbic acid analog substantially reverses the stable activity of ascorbic acid *in vitro* or *in vivo*.

In the present application, the term "effective amount" or "effective dose" generally refers to an amount that is sufficient to achieve or at least partially achieve the desired effect. "A therapeutically effective amount" or "a therapeutically effective dose" of a drug or therapeutic agent generally refers to any amount of drug facilitating disease regression (which is commonly demonstrated by the reduction in the severity of disease symptoms, the increase of frequency and duration in the asymptomatic period of disease, or the prevention of impairment or disability caused by the disease) when the drug or therapeutic agent is used alone or in combination with another therapeutic agent. "A prophylactically effective amount" or "a prophylactically effective dose" of drug generally refers to an amount of drug inhibiting the development or recurrence of disease when the drug is administered alone or in combination with another therapeutic agent to a subject at a risk of disease development or recurrence. Many methods that are known to those skilled in the art, e.g., in a human subject during clinical trials or an animal model system, or by measuring the activity of agent in an *in vitro* assay, can be used to estimate the capacity of a therapeutic or prophylactic agent to facilitate the disease regression or inhibit the disease development or recurrence.

In the present application, the term "treat/treatment" generally refers to prophylactically administering a combination to a healthy subject to prevent the occurrence of a disease or disorder. It can further comprise prophylactically administering a combination to a patient with a pre-allergic disease to be treated. "Prevention" does not require 100% elimination of the likelihood of a disease or condition, in other words, "prevention" generally refers to a reduction in the likelihood or degree of occurrence of a disease or condition in the presence of the administered combination.

In the present application, the term "treat/treating" generally refers to a clinical intervention for altering a natural process of the treated individual or cells during the clinical pathological process. It can comprise improving disease status, eliminating lesions, or improving prognosis.

In the present application, the term "administer/administration" generally refers to introducing the pharmaceutical combination via any introduction or delivery route into the body of the subject. Any method for contacting cells, organs, or tissues with the pharmaceutical combination that is known to persons skilled in the art can be used. It comprises, but is not limited to, intra-arterial, intranasal, intraperitoneal, intravenous, intramuscular, subcutaneous, transdermal or oral administration. Daily dose can be divided into one, two, or more doses in suitable form to be administered at one, two, or more time points during a certain period of time.

In the present application, the term "tumor" generally refers to a neoplasm or solid lesion formed by abnormal cell growth. In the present application, the tumors can be solid tumors or non-solid tumors. In certain embodiments, a tangible mass that can be detected by clinical examination, e.g., X-ray, CT scan, B-ultrasound, or palpation can be called a solid tumor, and a tumor that cannot be seen or detected by X-ray, CT scan, B-ultrasound or palpation can be called a non-solid tumor, e.g., leukemia.

In the present application, the term "subject" generally refers to a human or non-human animal, including, but not limited to, cat, dog, horse, pig, cow, sheep, rabbit, mouse, rat, or monkey, etc.

In the present application, the term "preparation", also known as pharmaceutical preparation, generally refers to a drug that is formulated in accordance with certain requirement of dosage form and can be provided to a subject for use to meet the requirements of treatment or prevention. For example, the preparation can comprise active ingredients and carriers.

In the present application, the term "carrier" generally refers to any other substance in addition to the active ingredients. For example, a pharmaceutically acceptable substance, composition, or vehicle involving carrying or transferring a chemical agent. For example, buffers, surfactants, stabilizers, preservatives, adsorption-promoting agents for enhancing the bioavailability, liquid or solid filling agents, diluents, excipients, solvents, coating materials, and/or other conventional co-solvents or dispersants, etc.

In the present application, the term "container" generally refers to any vessel or device suitable for holding a drug. For example, drug boxes, drug bottles, drug bags, blisters, tubes, syringes, etc.

In the present application, the term "comprise/comprising" or "include/including" generally refers to including explicitly specified features, but not excluding other elements.

In the present application, the term "about" generally refers to a variation within 0.5%-10% of the specified value, e.g., a variation within 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% of the specified value.

## Detailed Description

In one aspect, the present application provides a pharmaceutical combination comprising:

a) a prophylactically and/or therapeutically effective amount of ascorbic acid or a derivative thereof; and
b) a prophylactically and/or therapeutically effective amount of a second therapeutic agent selected from the group consisting of: a Notch signaling pathway inhibitor, an Hh signaling pathway inhibitor, a Porcupine inhibitor, an RXRα inhibitor, Niclosamide, a CK1a inhibitor, and a Frizzled receptor inhibitor.

In another aspect, the present application further provides use of a combination of ascorbic acid or a derivative thereof with a second therapeutic agent in the manufacture of a drug for preventing and/or treating tumors, wherein the second therapeutic agent is selected from the group consisting of: a Notch signaling pathway inhibitor, an HH signaling pathway inhibitor, an RXRα inhibitor, and a Wnt signaling pathway inhibitor.

For example, the second therapeutic agent can comprise a Notch signaling pathway inhibitor.

For example, the second therapeutic agent can comprise a SHH signaling pathway inhibitor.

For example, the second therapeutic agent can comprise an RXRα inhibitor.

For example, the second therapeutic agent can comprise a Wnt signaling pathway inhibitor.

In another aspect, the present application further provides a method for preventing and/or treating tumors comprising administering to a subject in need thereof:

a) ascorbic acid or a derivative thereof; and
b) a second therapeutic agent, wherein the second therapeutic agent is selected from the group consisting of: a Notch signaling pathway inhibitor, an HH signaling pathway inhibitor, an RXRα inhibitor, and a Wnt signaling pathway inhibitor.

In another aspect, the present application further provides a method for monitoring a response to a drug in a subject comprising:

a) administering to the subject ascorbic acid or a derivative thereof and a second therapeutic agent, wherein the second therapeutic agent is selected from the group consisting of: a Notch signaling pathway inhibitor, an HH signaling pathway inhibitor, an RXRα inhibitor, and a Wnt signaling pathway inhibitor; and
b) detecting a change in one or more of the level and/or activity of blood alkaline phosphatase, the level and/or activity of lactic dehydrogenase, the expression level of CADM1 gene, the expression level of CD44 gene, the expression level of livin gene, the expression level of HIF-1α gene, the expression level of ET-1 gene, the expression level of IGF-1R gene, the expression level of STAT3 gene, the expression level of CEA gene, the expression level of CA199 gene, the expression level of CA125 gene, the expression level of AFP gene, the expression level of CA724 gene, and the expression level of β-HCG gene in the subject after the administration of a).

In another aspect, the present application further provides a kit comprising the pharmaceutical combination as described in the present application.

## Pharmaceutical Combination

The pharmaceutical combination of the present application comprises: a) a prophylactically and/or therapeutically effective amount of ascorbic acid or a derivative thereof as described in the present application; and b) a prophylactically and/or therapeutically effective amount of a second therapeutic agent selected from the group consisting of: a Notch signaling pathway inhibitor, an HH signaling pathway inhibitor, a Porcupine inhibitor, an RXRα inhibitor, Niclosamide, a CK1a inhibitor, and a Frizzled receptor inhibitor as described in the present application. Of those, the ascorbic acid or a derivative thereof can comprise ascorbic acid, an ascorbic acid derivative, or any combination thereof. The second therapeutic agent can comprise any one or a combination of any several of a Notch signaling pathway inhibitor, HH signaling pathway inhibitor, Porcupine inhibitor, RXRα inhibitor, Niclosamide, CK1a inhibitor, and Frizzled receptor inhibitor.

For example, the second therapeutic agent can comprise a Notch signaling pathway inhibitor.

For example, the second therapeutic agent can comprise an HH signaling pathway inhibitor.

For example, the second therapeutic agent can comprise a Porcupine inhibitor.

For example, the second therapeutic agent can comprise an RXRα inhibitor.

For example, the second therapeutic agent can comprise a Niclosamide.

For example, the second therapeutic agent can comprise a CK1a inhibitor.

For example, the second therapeutic agent can comprise a Frizzled receptor inhibitor.

For example, the ratio of the effective amount of the second therapeutic agent to the effective amount of the ascorbic acid or a derivative thereof is about 1:1 to about 1:5000.

For example, the ratio of the effective amount of the second therapeutic agent to the effective amount of the ascorbic acid or a derivative thereof is about 1:10 to about 1:5000.

For example, the ratio of the effective amount of the second therapeutic agent to the effective amount of the ascorbic acid or a derivative thereof is 1:1 to about 1:20, about 1:5 to about 1:20, about 1:10 to about 1:20, about 1:10 to about 1:25, about 1:10 to about 1:50, about 1:10 to about 1:100, about 1:10 to about 1:150, about 1:10 to about 1:200, about 1:10 to about 1:250, about 1:10 to about 1:300, about 1:10 to about 1:400, about 1:10 to about 1:450, about 1:10 to about 1:500, about 1:10 to about 1:550, about 1:10 to about 1:600, about 1:10 to about 1:650, about 1:10 to about 1:700, about 1:10 to about 1:800, about 1:10 to about 1:900, about 1:10 to about 1:1000, about 1:10 to about 1:1500, about 1:10 to about 1:2000, about 1:10 to about 1:3000, about 1:10 to about 1:4000, about 1:10 to about 1:5000, about 1:5 to about 1:20, about 1:5 to about 1:25, about 1:5 to about 1:35, about 1:5 to about 1:50, about 1:5 to about 1:100, about 1:5 to about 1:150, about 1:5 to about 1:200, about 1:25 to about 1:50, about 1:25 to about 1:100, about 1:25 to about 1:150, about 1:25 to about 1:200, about 1:25 to about 1:1000, about 1:25 to about 1:5000, about 1:50 to about 1:100, about 1:50 to about 1:150, about 1:50 to about 1:200, about 1:50 to about 1:300, about 1:50 to about 1:500, about 1:50 to about 1:1000, about 1:50 to about 1:2000, about 1:50 to about 1:4000 or about 1:50 to about 1:5000, about 1:4500 to about 1:5500.

For example, the ratio of the effective amount of the second therapeutic agent to the effective amount of the ascorbic acid or a derivative thereof is about 1:1, 1:5, 1:7, 1:10, 1:12, 1:15, 1:17, 1:20, 1:25, 1:35, 1:40, 1:45, 1:50, 1:55, 1:60, 1:65, 1:70, 1:75, 1:80, 1:85, 1:90, 1:100, 1:120, 1:150, 1:180, 1:200, 1:220, 1:250, 1:280, 1:300, 1:320, 1:350, 1:380, 1:400, 1:420, 1:450, 1:480, 1:500, 1:550, 1:600, 1:650, 1:700, 1:750, 1:800, 1:850, 1:900, 1:950, 1:1000, 1:1100, 1:1200, 1:1300, 1:1400, 1:1500, 1:1600, 1:1700, 1:1800, 1:1900, 1:2000, 1:2100, 1:2200, 1:2300, 1:2400, 1:2500, 1:2600, 1:2700, 1:2800, 1:2900, 1:3000, 1:3100, 1:3200, 1:3300, 1:3400, 1:3500, 1:3600, 1:3700, 1:3800, 1:3900, 1:4000, 1:4100, 1:4200, 1:4300, 1:4400, 1:4500, 1:4600, 1:4700, 1:4800, 1:4900 or 1:5000.

For example, the ratio of effective amount can comprise a molar ratio of effective amount and/or a mass ratio of effective amount.

For example, a mass ratio of the second therapeutic agent and the ascorbic acid or a derivative thereof is about 5000:1 to about 1:5000.

For example, the mass ratio of the second therapeutic agent and the ascorbic acid or a derivative thereof is about 20:1 to 1:1, about 20:1 to about 5:1, about 20:1 to about 10:1, about 25:1 to about 10:1, about 50:1 to about 10:1, about 100:1 to about 10:1, about 150:1 to about 10:1, about 200:1 to about 10:1, about 250:1 to about 10:1, about 300:1 to about 10:1, about 400:1 to about 10:1, about 450:1 to about 10:1, about 10:1 to about 500:1, about 10:1 to about 550:1, about 10:1 to about 600:1, about 10:1 to about 650:1, about 700:1 to about 10:1, about 800:1 to about 10:1, about 900:1 to about 10:1, about 1000:1 to about 10:1, about 1500:1 to about 10:1, about 2000:1 to about 10:1, about 3000:1 to about 10:1, about 4000:1 to about 10:1, about 5000:1 to about 10:1, about 20:1 to about 5:1 about 1000:1 to about 50:1, about 2000:1 to about 50:1, about 3000:1 to about 50:1, about 4000:1 to about 50:1, about 5000:1 to about 1:50, about 20:1 to 1:20, about 20:1 to about 1:20, about 20:1 to about 1:25, about 25:1 to about 50:1, about 50:1 to about 1:100, about 100:1 to about 1:100, about 150:1 to about 1:100, about 200:1 to about 1:200, about 250:1 to about 1:500, about 300:1 to about 1:1000, about 400:1 to about 1:100, about 450:1 to about 1:500, about 10:1 to about 1:500, about 10:1 to about 1:550, about 10:1 to about 1: 600, about 10:1 to about 1: 650, about 700:1 to about 1:650, about 800:1 to about 1:800, about 900:1 to about 1:900, about 1000:1 to about 1:1000, about 1500:1 to about 1:1000, about 2000:1 to about 1:1500, about 3000:1 to about 1:500, about 4000:1 to about 1:2500, about 5000:1 to about 1:3500, about 20:1 to about 1:1500, about 1000:1 to about 1:2000, about 2000:1 to about 1:5000 about 1:1 to about 1:20, about 1:5 to about 1:20, about 1:10 to about 1:20, about 1:10 to about 1:25, about 1:10 to about 1:50, about 1:10 to about 1:100, about 1:10 to about 1:150, about 1:10 to about 1:200, about 1:10 to about 1:250, about 1:10 to about 1:300, about 1:10 to about 1:400, about 1:10 to about 1:450, about 1:10 to about 1:500, about 1:10 to about 1:550, about 1:10 to about 1:600, about 1:10 to about 1:650, about 1:10 to about 1:700, about 1:10 to about 1:800, about 1:10 to about 1:900, about 1:10 to about 1:1000, about 1:10 to about 1:1500, about 1:10 to about 1:2000, about 1:10 to about 1:3000, about 1:10 to about 1:4000, about 1:10 to about 1:5000, about 1:5 to about 1:20, about 1:5 to about 1:25, about 1:5 to about 1:35, about 1:5 to about 1:50, about 1:5 to about 1:100, about 1:5 to about 1:150, about 1:5 to about 1:200, about 1:25 to about 1:50, about 1:25 to about 1:100, about 1:25 to about 1:150, about 1:25 to about 1:200, about 1:25 to about 1:1000, about 1:25 to about 1:5000, about 1:50 to about 1:100, about 1:50 to about 1:150, about 1:50 to about 1:200, about 1:50 to about 1:300, about 1:50 to about 1:500, about 1:50 to about 1:1000, about 1:50 to about 1:2000, about 1:50 to about 1:3000, about 1:50 to about 1:4000 or about 1:50 to about 1:5000.

For example, a mass ratio of the second therapeutic agent and the ascorbic acid or a derivative thereof is about 1:10 to about 1:5000.

For example, the second therapeutic agent and the ascorbic acid or a derivative thereof can each be from different sources, e.g., prepared, produced or sold by different manufacturers. For example, the prepared, produced, or sold ascorbic acid or a derivative thereof are not required to be single-component or pure, as long as it contains a compound capable of releasing ascorbic acid (vitamin C) *in vivo* or *in vitro*, or solvates, hydrates, salts thereof, and/or it contains an ascorbic acid analog (e.g., an analog wherein one or more hydroxyl groups in ascorbic acid are replaced with another moiety, and wherein the ascorbic acid analog substantially reserves the stable activity of ascorbic acid *in vivo* or *in vitro*), all of which are encompassed within the scope of the present application. For the same reason, the prepared, produced, or sold second therapeutic agent is not required to be single-component or pure, as long as it contains a substance capable of releasing one or more of the second therapeutic agents as described in the present application *in vivo* and *in vitro*, or solvates, hydrates, salts thereof, and/or it contains analog(s) of any one or more of the second therapeutic agents as described in the present application (e.g., an analog which comprises a group modification or substitution, and substantially reserves the stable activity of any second therapeutic agent *in vitro* or *in vivo*), all of which are encompassed within the scope of the present application.

For example, the second therapeutic agent and the ascorbic acid or a derivative thereof are each present in different containers.

For example, the second therapeutic agent and the ascorbic acid or a derivative thereof are each present in different containers, while the amount of the second therapeutic agent and the amount of the ascorbic acid or a derivative thereof can or cannot be set in accordance with the effective amount ratio thereof.

For example, the second therapeutic agent can be present in 1 or more containers, e.g., 2 or more, 3 or more, 4 or more, 5 or more, 6 or more, 7 or more, 8 or more, 9 or more, 10 or more, 11 or more, 12 or more.

For example, the ascorbic acid or a derivative thereof can be present in 1 or more containers, e.g., 2 or more, 3 or more, 4 or more, 5 or more, 6 or more, 7 or more, 8 or more, 9 or more, 10 or more, 11 or more, 12 or more.

For example, the containers can comprise drug chests, drug boxes, drug bottles, drug bags, blisters, tubes, syringes, etc.; for example, the drug bottle can comprise sealed glass ampules, tubes, vials, flasks, bottles, etc.; for example, the containers can be made from glass, organic polymers like polycarbonates, polystyrenes, etc., or ceramics, metals, composite films, cellophanes, packing materials partially lined with aluminum foil, alloy foil, or the like, as well as any other suitable material commonly used for retaining an agent.

For example, the second therapeutic agent and the ascorbic acid or a derivative thereof can each be present in different containers, and be simultaneously or respectively formulated with a suitable carrier into preparation as required.

For example, the second therapeutic agent and the ascorbic acid or a derivative thereof are mixed and formulated into a suitable preparation.

For example, the second therapeutic agent and the ascorbic acid or a derivative thereof are each formulated to different suitable preparations.

For example, the second therapeutic agent and the ascorbic acid or a derivative thereof are each present in a form of preparation in different containers.

For example, the pharmaceutical combination comprises a first preparation and a second preparation, wherein the first preparation comprises the ascorbic acid or a derivative thereof and a pharmaceutically acceptable first carrier, and the second preparation comprises the second therapeutic agent and a pharmaceutically acceptable second carrier.

For example, the first preparation and the second preparation can be the same dosage form or can be different dosage forms.

For example, both the first preparation and the second preparation can be any one of pills, powders, tablets, granules, gels, hard capsules, soft capsules, syrups, mixtures, lotions, injections, aerosols, ointments, films, suppositories, drop pills, and the like, or can be a combination of any two different dosage forms of the above.

For example, the first preparation and the second preparation can each be present in preparation forms suitable for the same administration mode.

For example, the first preparation and the second preparation can each be present in preparation forms suitable for different administration modes.

For example, the pharmaceutical combination comprises a pharmaceutical composition, and the pharmaceutical composition comprises the second therapeutic agent and the ascorbic acid or a derivative thereof.

For example, the second therapeutic agent has a content of about 1% to about 50% (w/w) in the pharmaceutical composition.

About 5% (w/w) to about 50% (w/w), about 10% (w/w) to about 40% (w/w), about 20% (w/w) to about 50% (w/w), about 10% (w/w) to about 30% (w/w), about 20% (w/w) to about 40% (w/w), about 1% (w/w) to about 5% (w/w). About 30% (w/w) to about 50% (w/w), about 5% to about 20% (w/w), about 20% (w/w) to about 30% (w/w).

For example, the second therapeutic agent has a content of about 5% to about 20% (w/w) in the pharmaceutical composition.

For example, the second therapeutic agent has a content of about 5% (w/w) to about 10% (w/w), about 10% (w/w) to about 20% (w/w), about 10% (w/w) to about 15% (w/w), about 10% (w/w) to about 18% (w/w), about 5% (w/w) to about 15% (w/w).

For example, the ascorbic acid or a derivative thereof have a content of about 50% to 95% (w/w) in the pharmaceutical composition.

For example, the ascorbic acid or a derivative thereof has a content of about 50% (w/w) to about 60% (w/w), about 50% (w/w) to about 70% (w/w), about 60% (w/w) to about 80% (w/w), about 50% (w/w) to about 90% (w/w), about 60% (w/w) to about 90% (w/w), about 55% (w/w) to about 75% (w/w). About 70% (w/w) to about 90% (w/w), about 80 to about 95% (w/w).

For example, the ascorbic acid or derivatives thereof have a content of about 80% to 95% (w/w) in the pharmaceutical composition.

For example, the second therapeutic agent has a content of about 80% to about 85% (w/w), about 80% to about 90% (w/w), about 85% to about 95% (w/w), about 85% to about 90% (w/w).

## Use and Method

The present application further provides use of a combination of ascorbic acid or a derivative thereof with a second therapeutic agent in the manufacture of a drug for preventing and/or treating tumors, wherein the second therapeutic agent is selected from the group consisting of: a Notch signaling pathway inhibitor, an HH signaling pathway inhibitor, an RXRα inhibitor, and a Wnt signaling pathway inhibitor.

The present application further provides a method for preventing and/or treating tumors comprising administering to a subject in need thereof:

a) ascorbic acid or a derivative thereof; and

b) a second therapeutic agent, wherein the second therapeutic agent is selected from the group consisting of: a Notch signaling pathway inhibitor, an HH signaling pathway inhibitor, an RXRα inhibitor, and a Wnt signaling pathway inhibitor.

The present application further provides a method for monitoring a response to a drug in a subject comprising:

a) administering to the subject ascorbic acid or a derivative thereof and a second therapeutic agent, wherein the second therapeutic agent is selected from the group consisting of: a Notch signaling pathway inhibitor, an HH signaling pathway inhibitor, an RXRα inhibitor, and a Wnt signaling pathway inhibitor; and

b) detecting a change in one or more of the level and/or activity of blood alkaline phosphatase, the level and/or activity of lactic dehydrogenase, the expression level of CADM1 gene, the expression level of CD44 gene, the expression level of livin gene, the expression level of HIF-1α gene, the expression level of ET-1 gene, the expression level of IGF-1R gene, the expression level of STAT3 gene, the expression level of CEA gene, the expression level of CA199 gene, the expression level of CA125 gene, the expression level of AFP gene, the expression level of CA724 gene, and the expression level of β-HCG gene in the subject after the administration of a).

For example, the expression level of gene can comprise the modification and/or mutation of genomic DNA, the expression level of mRNA, and the expression level of protein, e.g., the detection can be selected from the group consisting of PCR, real-time quantitative PCR, digital PCR, liquid chip, solid chip, hybridization in situ, normal sequencing and high-throughput sequencing for detecting the modification and/or mutation of genomic DNA, and the expression level of mRNA.

For example, the detection can be selected from the group consisting of liquid chip, solid chip, ELISA, radio-immunity, chemiluminescence immune assay, mass spectrum, HPLC, Western blotting and sequencing for detecting the expression level of protein.

For example, the ascorbic acid or a derivative thereof can comprise ascorbic acid, an ascorbic acid derivative, or any combination thereof. The second therapeutic agent can comprise any one or a combination of any several of a Notch signaling pathway inhibitor, an HH signaling pathway inhibitor, and a Wnt signaling pathway inhibitor RXRα.

For example, the second therapeutic agent can comprise a Notch signaling pathway inhibitor.

For example, the second therapeutic agent can comprise an HH signaling pathway inhibitor.

For example, the second therapeutic agent can comprise an RXRα inhibitor.

For example, the second therapeutic agent can comprise an HH signaling pathway inhibitor.

For example, the tumors comprise solid tumors or non-solid tumors.

In some embodiments, the solid tumors can be selected from the group consisting of: liver cancer, stomach cancer, lung cancer, breast cancer, colon cancer, rectal cancer, renal cell carcinoma, liver cancer, non-small cell lung cancer, small intestine cancer, esophagus cancer, melanoma, osteosarcoma, pancreatic cancer, skin cancer, head or neck cancer, cutaneous or intraocular malignant melanoma, uterus cancer, ovarian cancer, rectal cancer, anal cancer, stomach cancer, testis cancer, fallopian tube carcinoma, endometrial cancer, cervical cancer, vaginal cancer, vulval cancer, Hodgkin's disease, non-Hodgkin's lymphoma, carcinoma of endocrine system, thyroid cancer, parathyroid cancer, adrenal carcinoma, soft tissue sarcoma, urethral carcinoma, carcinoma of penis, pediatric solid tumor, bladder cancer, renal and ureteral cancer, carcinoma of renal pelvis, central nervous system (CNS) tumor, primary CNS lymphoma, tumor angiogenesis, spinal tumor, brainstem glioma, pituitary adenoma, Kaposi sarcoma, epidermoid, squamous cell carcinoma, T cell lymphoma, and a metastatic lesion of the cancers.

For example, the non-solid tumors can be selected from the group consisting of: chronic lymphoblastic leukemia (CLL), acute leukemia, acute lymphoblastic leukemia (ALL), B cell acute lymphoblastic leukemia (B-ALL), chronic myeloid leukemia (CML), acute myeloid leukemia (AML), B cell prolymphocytic leukemia, blast cell plasmacytoid dendritic cytoma, Burkitt's lymphoma, diffuse large B cell lymphoma, follicular lymphoma, hairy cell leukemia, small or large cell follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablast lymphoma, plasmacytoiddentritic cytoma, and Waldenstrom macroglobulinemia.

For example, the solid tumors comprise osteosarcoma and/or colon cancer.

For example, the non-solid tumors comprise lymphoma.

For example, the second therapeutic agent and the ascorbic acid or a derivative thereof are formulated to be simultaneously administered to the subject.

For example, the Niclosamide in combination with the ascorbic acid or a derivative thereof can be used for used for preventing and/or treating tumors. For example, the Niclosamide in combination with the ascorbic acid or a derivative thereof can be used for preventing and/or treating lymphoma, osteosarcoma, and/or colon cancer. For example, the Dvl-2 inhibitor in combination with the ascorbic acid or a derivative thereof can be used for preventing and/or treating lymphoma, osteosarcoma and/or colon cancer. For example, the Wnt signaling pathway inhibitor in combination with the ascorbic acid or a derivative thereof can be used for preventing and/or treating lymphoma, osteosarcoma, and/or colon cancer.

For example, the simultaneous administration to the subject can comprise administering the second therapeutic agent and the ascorbic acid or a derivative thereof to the subject at a time interval of no more than 1 hr. For example, the time interval is 60 min, 55 min, 50 min, 45 min, 40 min, 35 min, 30 min, 25 min, 20 min, 15 min, 10 min, 8 min, 5 min, 3 min, 2 min, 1 min, or the ingredients are administered together in a mixed form.

For example, the second therapeutic agent and the ascorbic acid or a derivative thereof are formulated to be separately administered to the subject.

For example, the separate administration to the subject can comprise administering the second therapeutic agent and the ascorbic acid or a derivative thereof to the subject at a time interval of more than 1 hr. For example, the interval is 1.5 hrs., 2 hrs., 2.5 hrs., 3 hrs., 3.5 hrs., 4 hrs., 4.5 hrs., 5 hrs., 5.5 hrs., 6 hrs., 6.5 hrs., 7 hrs., 7.5 hrs., 8 hrs., 9 hrs., 10 hrs., 11 hrs., 12 hrs., 15 hrs., 18 hrs., 21 hrs., 24 hrs., 2 days, 3 days, 4 days, 5 days, 6 days, 7 days, or longer.

For example, the administration can comprise any one of oral administration, intravenous administration, intramuscular administration, *in situ* administration at tumor site, inhalation, rectal administration, vaginal administration, transdermal administration, or subcutaneous depot administration, or any combination thereof.

For example, the separate administration to the subject can comprise alternately administering the second therapeutic agent and the ascorbic acid or a derivative thereof, e.g., administering the second therapeutic agent, and then administering the ascorbic acid or a derivative thereof, or administering the ascorbic acid or a derivative thereof, and then administering the second therapeutic agent.

For example, the administration of the second therapeutic agent can comprise 1 administration.

For example, the administration of the second therapeutic agent can comprise 2 or more continuous administrations, e.g., 3 or more, 4 or more, 5 or more, 6 or more, 7 or more, 8 or more, 9 or more, 10 or more.

For example, the administration of the ascorbic acid or a derivative thereof can comprise 1 administration.

For example, the administration of the ascorbic acid or a derivative thereof can comprise 2 or more continuous administrations, e.g., 3 or more, 4 or more, 5 or more, 6 or more, 7 or more, 8 or more, 9 or more, 10 or more.

For example, the drug is formulated so that the second therapeutic agent is administered at a dose of about 1 mg/kg to about 100 mg/kg.

For example, the second therapeutic agent is administrated at a dose of about 10 mg/kg to about 100 mg/kg, 10 mg/kg to about 80 mg/kg, 10 mg/kg to about 50 mg/kg, 20 mg/kg to about 100 mg/kg, 30 mg/kg to about 80 mg/kg, 1mg/kg to about 30 mg/kg, 1mg/kg to about 20 mg/kg, about 1mg/kg to about 5mg/kg, 5mg/kg to about 10 mg/kg, 3mg/kg to about 8mg/kg, 5mg/kg to about 30 mg/kg, 30 mg/kg to about 70 mg/kg, 40 mg/kg to about 60 mg/kg, 20 mg/kg to about 50 mg/kg, 30 mg/kg to about 60 mg/kg, 20 mg/kg to about 70 mg/kg.

For example, the administration subject is a mouse. For example, the drug is formulated so that the second therapeutic agent is administered at a dose of about 10mg/kg to about 100 mg/kg.

For example, the administration subject is human. For example, the drug is formulated so that the second therapeutic agent is administered at a dose of about 1 mg/kg to about 10mg/kg.

For example, the drug is formulated so that the ascorbic acid or a derivative thereof is administered at a dose of about 0.05 g/kg to about 2.5 g/kg.

For example, the ascorbic acid or a derivative thereof is administered at a dose of about 0.05 g/kg to about 0.1 g/kg, about 0.1 g/kg to about 0.2 g/kg, about 0.15 g/kg to about 0.25 g/kg, about 0.05 g/kg to about 0.15 g/kg, about 0.05 g/kg to about 2.5 g/kg, about 0.25 g/kg to about 1 g/kg, about 0.35 g/kg to about 0.5 g/kg, about 0.5 g/kg to about 1 g/kg, about 0.5 g/kg to about 2.5 g/kg, about 0.5 g/kg to about 2 g/kg, about 1 g/kg to about 2 g/kg, about 1 g/kg to about 2.5 g/kg, about 1.5 g/kg to about 2.5 g/kg, about 0.1 g/kg to about 2.5 g/kg, about 0.5 g/kg to about 2 g/kg, about 0.05 g/kg to about 0.2 g/kg.

For example, the administration subject is a mouse. For example, the drug is formulated so that the ascorbic acid or a derivative thereof is administered at a dose of about 0.5g/kg to about 2.5 g/kg.

For example, the administration subject is human. For example, the drug is formulated so that the ascorbic acid or a derivative thereof is administered at a dose of about 0.05 g/kg to about 0.25g/kg.

For example, the drug is formulated so that the second therapeutic agent and the ascorbic acid or a derivative thereof are administered at a mass ratio of 1:5 to about 1:5000.

For example, the drug is formulated so that the second therapeutic agent and the ascorbic acid or a derivative thereof are administered at a mass ratio of about 1:1 to about 1:20, about 1:5 to about 1:20, about 1:10 to about 1:20, about 1:10 to about 1:25, about 1:10 to about 1:50, about 1:10 to about 1:100, about 1:10 to about 1:150, about 1:10 to about 1:200, about 1:10 to about 1:250, about 1:10 to about 1:300, about 1:10 to about 1:400, about 1:10 to about 1:450, about 1:10 to about 1:500, about 1:10 to about 1:550, about 1:10 to about 1:600, about 1:10 to about 1:650, about 1:10 to about 1:700, about 1:10 to about 1:800, about 1:10 to about 1:900, about 1:10 to about 1:1000, about 1:10 to about 1:1500, about 1:10 to about 1:2000, about 1:10 to about 1:3000, about 1:10 to about 1:4000, about 1:10 to about 1:5000, about 1:5 to about 1:20, about 1:5 to about 1:25, about 1:5 to about 1:35, about 1:5 to about 1:50, about 1:5 to about 1:100, about 1:5 to about 1:150, about 1:5 to about 1:200, about 1:25 to about 1:50, about 1:25 to about 1:100, about 1:25 to about 1:150, about 1:25 to about 1:200, about 1:25 to about 1:1000, about 1:25 to about 1:5000, about 1:50 to about 1:100, about 1:50 to about 1:150, about 1:50 to about 1:200, about 1:50 to about 1:300, about 1:50 to about 1:500, about 1:50 to about 1:1000, about 1:50 to about 1:2000, about 1:50 to about 1:4000 or about 1:50 to about 1:5000, about 1:4500 to about 1:5000.

For example, the drug is formulated so that the second therapeutic agent and the ascorbic acid or a derivative thereof at a mass ratio of about 1:5 to about 1:100.

For example, the drug is formulated so that the second therapeutic agent and the ascorbic acid or a derivative thereof are administered at a mass ratio of about 1:5 to about 1:20, about 1:10 to about 1:20, about 1:10 to about 1:25, about 1:10 to about 1:50, about 1:10 to about 1:100, about 1:5 to about 1:25, about 1:5 to about 1:35, about 1:5 to about 1:50, about 1:30 to about 1:80, about 1:40 to about 1:80, about 1:20 to about 1:100, about 1:20 to about 1:60, about 1:20 to about 1:50, about 1:5 to about 1:100, about 1:25 to about 1:50, about 1:25 to about 1:100, about 1:50 to about 1:100.

For example, the drug is formulated so that the second therapeutic agent and the ascorbic acid or a derivative thereof is administered at an effective amount ratio of about 1:5 to about 1:5000.

For example, the drug is formulated so that the second therapeutic agent and the ascorbic acid or a derivative thereof are administered at an effective amount ratio of about 1:1 to about 1:20, about 1:5 to about 1:20, about 1:10 to about 1:20, about 1:10 to about 1:25, about 1:10 to about 1:50, about 1:10 to about 1:100, about 1:10 to about 1:150, about 1:10 to about 1:200, about 1:10 to about 1:250, about 1:10 to about 1:300, about 1:10 to about 1:400, about 1:10 to about 1:450, about 1:10 to about 1:500, about 1:10 to about 1:550, about 1:10 to about 1:600, about 1:10 to about 1:650, about 1:10 to about 1:700, about 1:10 to about 1:800, about 1:10 to about 1:900, about 1:10 to about 1:1000, about 1:10 to about 1:1500, about 1:10 to about 1:2000, about 1:10 to about 1:3000, about 1:10 to about 1:4000, about 1:10 to about 1:5000, about 1:5 to about 1:20, about 1:5 to about 1:25, about 1:5 to about 1:35, about 1:5 to about 1:50, about 1:5 to about 1:100, about 1:5 to about 1:150, about 1:5 to about 1:200, about 1:25 to about 1:50, about 1:25 to about 1:100, about 1:25 to about 1:150, about 1:25 to about 1:200, about 1:25 to about 1:1000, about 1:25 to about 1:5000, about 1:50 to about 1:100, about 1:50 to about 1:150, about 1:50 to about 1:200, about 1:50 to about 1:300, about 1:50 to about 1:500, about 1:50 to about 1:1000, about 1:50 to about 1:2000, about 1:50 to about 1:4000 or about 1:50 to about 1:5000, about 1:4500 to about 1:5000.

For example, the drug is formulated so that the second therapeutic agent and the ascorbic acid or a derivative thereof are administered at an effective amount ratio of about 1:5 to about 1:100.

For example, the drug is formulated so that the second therapeutic agent and the ascorbic acid or a derivative thereof are administered at an effective amount ratio of about 1:5 to about 1:20, about 1:10 to about 1:20, about 1:10 to about 1:25, about 1:10 to about 1:50, about 1:10 to about 1:100, about 1:5 to about 1:25, about 1:5 to about 1:35, about 1:5 to about 1:50, about 1:30 to about 1:80, about 1:40 to about 1:80, about 1:20 to about 1:100, about 1:20 to about 1:60, about 1:20 to about 1:50, about 1:5 to about 1:100, about 1:25 to about 1:50, about 1:25 to about 1:100, about 1:50 to about 1:100.

## Inhibitors

In the present application, the Notch signaling pathway inhibitor, the HH signaling pathway inhibitor, the RXRα inhibitor, and the Wnt signaling pathway inhibitor can comprise a small molecular compound.

For example, the Notch signaling pathway inhibitor can comprise CB-103, FLI-06, JI051, PsoralidinTangeretin, Carvacrol, Notch inhibitor 1, BMS-906024, IMR-1, IMR-1A, Z-Ile-Leu-aldehyde, ZLDI-8, Semagacestat, Bruceine D, RO4929097, LY-411575, YO-01027, Crenigacestat, Avagacestat, Z-Ile-Leu-aldehyde, MK0752 and/or PF-03084014.

For example, the Notch signaling pathway inhibitor can comprise a γ-secretase (Gamma Secretase) inhibitor, an ADAM-17 inhibitor, a Notch ligand inhibitor, a Notch receptor inhibitor, and/or a CSL-DNA binding protein inhibitor.

For example, the Notch signaling pathway inhibitor can comprise a Notch ligand inhibitor.

For example, the Notch ligands inhibitor can comprise a Delta-like 1 inhibitor, a Delta-like 3 inhibitors, a Delta-like 4 inhibitor, a Jagged 1 inhibitor, and/or a Jagged 2 inhibitor.

For example, the Jagged 1 inhibitor can comprise Carvacrol.

For example, the Notch signaling pathway inhibitor can comprise a Notch receptor inhibitor.

For example, the Notch receptor inhibitor can comprise a Notch1 receptor inhibitor, a Notch2 receptor inhibitor, a Notch3 receptor inhibitor, and/or a Notch4 receptor inhibitor.

For example, the Notch receptor inhibitor can comprise Tangeretin, Carvacrol, Notch inhibitor 1, BMS-906024, IMR-1, IMR-1A, Z-Ile-Leu-aldehyde, Semagacestat and/or Bruceine D.

For example, the Notch1 receptor inhibitor can comprise Tangeretin, Carvacrol and/or a Notch inhibitor 1.

For example, the Notch3 receptor inhibitor can comprise a Notch inhibitor 1.

For example, the ADAM-17 inhibitor can comprise ZLDI-8.

For example, the Notch signaling pathway inhibitor comprises a γ-secretase inhibitor.

For example, the γ-secretase (Gamma Secretase) inhibitor can comprise RO4929097, LY-411575, YO-01027, Crenigacestat, Semagacestat, Avagacestat, Z-Ile-Leu-aldehyde, MK0752 and/or PF-03084014.

For example, the γ-secretase inhibitor comprises MK0752, RO4929097 and/or PF-03084014.

For example, the HH signaling pathway inhibitor can comprise Jervine, Ciliobrevin D, RU-SKI43 hydrochloride, RU-SKI43, JK184, Cyclopamine, Mebendazole, CiliobrevinA, SANT-1, Vismodegib, GANT 61, HhAntag, Saridegib, MRT-83, PF-5274857, 20(S)-Hydroxycholesterol, CUR61414, MK-4101, Vismodegib, Sonidegib and/or Glasdegib.

For example, the HH signaling pathway inhibitor can comprise a Hedgehog inhibitor, a Gli inhibitor, and/or a smo inhibitor.

For example, the HH signaling pathway inhibitor can comprise a Hedgehog inhibitor.

For example, the Hedgehog inhibitor can comprise Jervine, Ciliobrevin D, RU-SKI 43 hydrochloride, RU-SKI 43, JK184, Cyclopamine, Mebendazole, Ciliobrevin A, SANT-1, Vismodegib, CUR61414, MK-4101 and/or Dynarrestin.

For example, the HH signaling pathway inhibitor can comprise a Gli inhibitor.

For example, the Gli inhibitor can comprise GANT 61 and/or HhAntag.

For example, the Smo inhibitor can comprise Saridegib, MRT-83, Jervine, SANT-1, PF-5274857, 20(S)-Hydroxycholesterol, CUR61414, MK-4101, Vismodegib, Sonidegib and/or Glasdegib.

For example, the HH signaling pathway inhibitor comprises an SMO inhibitor.

For example, the SMO inhibitor can comprise Vismodegib, Sonidegib and/or Glasdegib.

For example, the Wnt signaling pathway inhibitor can comprise a Tankyrases inhibitor, a Porcupine inhibitor, a Dvl-2 inhibitor, a DKK inhibitor, a CK1a inhibitor, and/or a Frizzled receptor inhibitor.

For example, the Wnt signaling pathway inhibitor can comprise a Porcupine inhibitor, a Dvl-2 inhibitor, CK1a inhibitor, and/or a Frizzled receptor inhibitor.

For example, the Wnt signaling pathway inhibitor can comprise a Tankyrases inhibitor.

For example, the Tankyrases inhibitor can comprise IWR1, XAV939, NVP-TNKS656 and/or JW74.

For example, the Wnt signaling pathway inhibitor can comprise a Porcupine inhibitor.

For example, the Porcupine inhibitor can comprise IWP-2, LGK974 and/or ETC-159.

For example, the Porcupine inhibitor can comprise LGK974 and/or ETC-159.

For example, the Wnt signaling pathway inhibitor can comprise a DKK inhibitor.

For example, the DKK inhibitor can comprise DKN-01.

For example, the Wnt signaling pathway inhibitor can comprise a CK1a inhibitor.

For example, the CK1a inhibitor can comprise Pyrvinium.

For example, the Wnt signaling pathway inhibitor can comprise a Dvl-2 inhibitor.

For example, the Dvl-2 inhibitor can comprise NSC668036, J01-017a, Niclosamide and/or Sulindac.

For example, the Dvl-2 inhibitor can comprise Niclosamide and/or Sulindac.

For example, the Dvl-2 inhibitor cannot comprise Sulindac.

For example, the Wnt signaling pathway inhibitor can comprise a Frizzled receptor inhibitor.

In certain embodiments, the RXRα inhibitor comprises berberine or a pharmaceutically acceptable salt thereof.

For example, the pharmaceutically acceptable salt can comprise a salt formed by berberine with an organic acid, such as, any one or more of R-(+)-alpha-organic acid, hydroxycitric acid, eicosapentaenoic acid, docosahexaenoic acid, ursolic acid, corosolic acid, cinnamic acid, cholic acid, obeticholic acid, ursodeoxycholic acid, oleanolic acid, salicylic acid, betulinic acid, chlorogenic acid, caffeic acid, bassic acid, acetyl-L-carnitine, S-allylcysteine sulfoxide, S-methylcysteine sulfoxide, pantothenic acid, ascorbic acid, retinoic acid, niacin, and biotin.

For example, the inhibitor can further comprise Fervenulin, 3-Methyltoxoflavin and/or Walrycin B.

In the present application, the Notch signaling pathway inhibitor, the HH signaling pathway inhibitor, the RXRα inhibitor, and the Wnt signaling pathway inhibitor can further comprise an antibody or polypeptide. The interaction between the antibody or polypeptide and the corresponding binding target thereof can reduce or block the activation/signal transduction of the Notch signaling pathway, the HH signaling pathway, and the Wnt signaling pathway, and/or reduce or block the activity of the RXRα.

For example, the antibody or polypeptide can comprise an antibody or binding polypeptide of a γ-secretase (Gamma Secretase), an antibody or binding polypeptide of ADAM-17, an antibody or binding polypeptide of a Notch ligand, an antibody or binding polypeptide of a Notch receptor, an antibody or binding polypeptide of a CSL-DNA binding protein, an antibody or binding polypeptide of Hedgehog, an antibody or binding polypeptide of Gli, an antibody or binding polypeptide of smo, an antibody or binding polypeptide of Tankyrases, an antibody or binding polypeptide of Porcupine, an antibody or binding polypeptide of Dvl-2, an antibody or binding polypeptide of DKK, an antibody or binding polypeptide of CK1a, and/or an antibody or binding polypeptide of Frizzled receptor.

For example, the antibody of a Frizzled receptor can comprise OMP 18RS (Vantictumab), OMP-54F28 (Ipafricept), OMP131R10 and/or OTSA 101.

For example, the binding polypeptide of a Frizzled receptor can comprise Fz7-21 TFA, Fz7-21 and/or Foxy-5.

In the present application, the Notch signaling pathway inhibitor, the HH signaling pathway inhibitor, the RXRα inhibitor, and the Wnt signaling pathway inhibitor can further comprise an siRNA, shRNA and/or CRISPR/Cas9 system. The siRNA, shRNA and CRISPR/Cas9 system can target an encoding genomic DNA or mRNA of a molecule contained in the HH signaling pathway, the RXRα inhibitor, and the Wnt signaling pathway so that the expression level/activity of the corresponding molecule is reduced.

For example, the siRNA, shRNA and/or CRISPR/Cas9 system can comprise an siRNA, shRNA and/or CRISPR/Cas9 system targeting a γ-secretase (Gamma Secretase), ADAM-17, a Notch ligand, a Notch receptor, a CSL-DNA binding protein, Hedgehog, Gli, Smo, Tankyrases, Porcupine, Dvl-2, DKK, CK1a and/or a Frizzled receptor.

For example, the CRISPR/Cas9 system comprises a guide RNA and/or Cas9 protein .

## Ascorbic Acid and Derivative thereof

In the present application, the ascorbic acid or a derivative thereof comprises one selected from the group consisting of ascorbic acid (vitamin C), a derivative of ascorbic acid and any combination thereof.

For example, the ascorbic acid derivative can further comprise dehydroascorbic acid, an ascorbate or a salt of ascorbic acid derivative.

Ascorbic acid is sensitive to the influence of environmental parameters (e.g., light, heat, oxygen) due to its α-ketolactone structure. It may be unstable in water or other aqueous solution. Ascorbic acid derivatives that have a higher stability than the parent compound are prepared by means of chemically stabilizing the ascorbic acid molecule (e.g., see the U.S. Patents US 5,137,723 and US 5,078,989), the content of which is incorporated into the present application by reference.

For example, the derivative of ascorbic acid can be an ascorbic acid analog. Representative ascorbic acid derivatives comprise those in which at least one hydroxyl group (e.g., 2-OH, 3-OH, 5-OH, 6-OH) in the ascorbic acid molecule is derived with a modification group (e.g., the U.S. Patent US 5,078,989). For example, one or more of the hydroxyl groups can be substituted with another moiety. For example, the ascorbic acid and a derivative thereof comprise ascorbic acid and at least one ascorbic acid derivative.

For example, the ascorbic acid derivative can comprise free 2-OH and free 3-OH.

For example, the ascorbic acid derivative comprise an ascorbic acid ester derived from at least one of 5-OH and 6-OH.

For example, the ascorbic acid derivatives comprise an ester, e.g., 6-O-octanoyl-ascorbic acid, 6-O-dodecanoyl-ascorbic acid, 6-O-tetradecanoyl-ascorbic acid, 6-O-octodecanoyl-ascorbic acid, 6-O-dodecanedioyl-ascorbic acid, 6-O-decosanedioyl-ascorbic acid, 6-O-thapsoyl- ascorbic acid, 6-O-decanedioyl-ascorbic acid, 6-O-hexanedioyl-ascorbic acid.

For example, the ascorbic acid derivatives can further comprise esters in which the lipophilic moiety of the molecule is a mono- or poly-unsaturated fatty acid. For example, the unsaturated fatty acid can comprise health-associated essential fatty acids, e.g., $\omega$-3 ($\alpha$-linolenic acid), $\omega$-6 or $\omega$-9 fatty acid. For example, it can further comprise esters containing amino acid residue(s).

For example, the ascorbic acid derivatives can further comprise 2-O-alkyl or 3-O-alkyl derivatives of ascorbic acid. The 3-O-alkyl-ascorbic acid is reported by Nihro et al., in Chem. Pharm. Bull. 1991, 39: 1731-1735, which is incorporated into the present application by reference.

For example, the ascorbic acid derivative can comprise glycosides of ascorbic acid; such as, ascorbic acid 1-glucoside, ascorbic acid 2-glucoside, ascorbic acid 3-glucoside, ascorbic acid 5-glucoside and ascorbic acid 6-glucoside.

For example, the ascorbic acid derivative can comprise 2-O-($\alpha$-D-pyranoglucosyl)-ascorbic acid (see, e.g., the U.S. Patent US 5,137,723) and 2-O-($\beta$-D-pyranoglucosyl)- ascorbic acid (see, e.g., the U.S. Patent Application No. US 2005/0113312). The above-listed literatures are incorporated into the present application by reference.

For example, the ascorbic acid derivative can comprise a dual-functionalized derivative, such as, e.g., 6-O-acyl-2-O-($\alpha$-D-pyranoglucosyl)ascorbic acid (see, e.g., Yamamoto et. al, J.Med.Chem.2002, 45(2): 462-468. This literature is incorporated into the present application by reference.

For example, the ascorbic acid derivative can comprise an ascorbyl phosphate. For example, the ascorbyl phosphate is an alkali metal salt, an alkali earth metal salt, or a transition metal salt. Examples can comprise magnesium ascorbyl phosphate, sodium ascorbyl phosphate (e.g., sodium ascorbyl-2-monophosphate), calcium ascorbyl phosphate, potassium ascorbyl phosphate, and mixed salts, e.g., sodium magnesium ascorbyl phosphate, sodium calcium ascorbyl phosphate, aminopropyl ascorbyl phosphate.

For example, the ascorbyl phosphate can be present as a hydrate, commonly a dihydrate. A representative dihydrate can be purchased from DSM under the product name of STAY-C50, for example.

In the present application, the ascorbic acid derivative comprises a pharmaceutically acceptable ascorbate.

For example, the pharmaceutically acceptable ascorbate comprises an alkali metal salt, such as, sodium salt, potassium salt, etc.; an alkali earth metal salt, such as, calcium salt, magnesium salt, etc.; an alkali amino acid salt, such as, arginine salt or the like; and an organic amine salt, such as, triethanolamine salt, etc.

For example, the salt of ascorbic acid or ascorbic acid derivative can be an edible (e.g., pharmaceutically acceptable) salt, such as, calcium, sodium, magnesium, potassium, and zinc salts, as well as a mixed salt of ascorbic acid or ascorbic acid derivative.

For example, the pharmaceutically acceptable ascorbate comprises sodium ascorbate.

Other well-recognized ascorbic acid derivatives can also be used for the purpose of the present application.

## Carrier and Preparation

In the present application, the dosage forms of the first preparation, the second preparation or the pharmaceutical composition can comprise tablet, capsule, granule, powder, syrup, suspension, suppository, ointment, cream, gel, patch, inhalation, injection, etc. These preparations can be prepared by conventional methods.

For example, in the case of liquid preparations, they can be in a form of solution or suspension in water or another other suitable solvent in use.

For example, the tablet and the granule can be coated by well-known methods.

For example, in the case of injections, the active ingredients (including the ascorbic acid or a derivative thereof as described in the present application and the second therapeutic agent as described in the present application) can be dissolved in water to prepare the preparation, or can be dissolved in normal saline or dextrose solution as required. For example, a buffer or preservative can also be added.

For example, those can also be provided in any preparation form for oral administration or non-oral administration. For example, those can be prepared as a preparation for oral administration, such as, granule, fine granule, powder, hard capsule, soft capsule, syrup, emulsion, suspension or liquid, or the like.

For example, those can also be prepared as a preparation for non-oral administration, such as, injection for intravenous, intramuscular or subcutaneous administration, infusion, transdermal absorption preparation, transmucosal absorption preparation, nose drop, inhalation preparation, suppository, etc.

For example, the injection, infusion or the like can be prepared as a powdered dosage form such as lyophilized powder, which is used by dissolving in an appropriate aqueous medium such as normal saline in use.

For example, a sustained-release preparation coated with a polymer or the like can be directly administered into an organ such as the brain.

Persons skilled in the art can properly select the type of additive for preparation (carrier), the ratio of the additive for preparation (carrier) to the active ingredients, or the method for preparing the preparation used in the manufacture of the pharmaceutical preparation in accordance with the form of the preparation. For example, an inorganic or organic substance, or a solid or liquid substance can be used as additive for preparation (carrier), e.g., in an amount of 1-90% by weight relative to the weight of the active ingredient. For example, the carrier can comprise lactose, dextrose, mannitol, dextrin, cyclodextrin, starch, sucrose, aluminum magnesium metasilicate, synthetic aluminum silicate, sodium carboxymethylcellulose, hydroxypropyl starch, calcium carboxymethylcellulose, ion exchange resin, methylcellulose, gelatin, gum Arabic, hydroxypropylcellulose, hydroxypropyl methylcellulose, polyvinylpyrrolidone, polyvinyl alcohol, light anhydrous silicic acid, magnesium stearate, talc, tragacanth gum, bentonite, propolis, titania, sorbitan fatty acid ester, sodium lauryl sulfate, glycerin, glyceryl fatty Acid, purified lanolin, glycerogelatin, polysorbate, polyethylene glycol, vegetable oil, wax, liquid paraffin, white petrolatum, fluorocarbon, non-ionic surfactant, propylene glycol, water, and the like.

For example, during the manufacture of solid preparation for oral administration, the active ingredients (including ascorbic acid or derivatives thereof as described in the present application and the second therapeutic agent as described in the present application) can be mixed with the carrier ingredients (e.g., lactose, starch, crystalline cellulose, calcium lactate, anhydrous silicic acid) to prepare a powder. For example, binders such as sucrose, hydroxylpropylcellulose, polyvinylpyrrolidone or the like and disintegrants such as hydroxylmethylcellulose, calcium hydroxylmethylcellulose or the like can be further added, and the mixture is subject to dry or wet pelleting to prepare granules. For example, in the manufacture of tablets, the powders and/or granules can be subject to direct tableting. For example, lubricants such as magnesium stearate, talc powder, or the like can be further added for tableting. For example, the granules or tablets can also be coated with an enteric base such as hydroxylpropylmethylcellulose phthalate, methacrylic acid-methyl methacrylate polymer, or the like to prepare enteric preparations. For example, those can also be coated with ethylcellulose, carnauba wax, hydrogenated oil or the like to prepare a prolonged action preparation. For example, in the manufacture of capsules, powders or granules can be filled in a hard capsule. For example, the active ingredients can be coated with a gelatin film directly or after dissolved in glycerin, polyethylene glycol, sesame oil, olive oil or the like to prepare a soft capsule.

For example, in the manufacture of injections, the active ingredients can be dissolved together with pH adjusters such as hydrochloric acid, sodium hydroxide, lactose, lactic acid, sodium lactate, disodium hydrogen phosphate or the like and isotonic agents such as sodium chloride, dextrose, or the like in distilled water for injection. For example, those can be further subject to sterile filtration and filled in ampules. For example, mannitol, dextrin, cyclodextrin, gelatin or the like can be further added, and the mixture is vacuum lyophilized to prepare a ready-to-use injection. For example, lecithin, polysorbate 80, polyoxyethylene hydrogenated castor oil or the like can be added to the active ingredients, and the mixture can be emulsified in water to prepare an emulsion for injection.

For example, in the manufacture of preparations for rectal administration, the active ingredients can be moistened and dissolved together with a base for suppositories such as cocoa butter, triglyceride fatty acids, diglyceride fatty acids, monoglyceride fatty acid, polyethylene glycol, and the like, and then poured into a mold for cooling. For example, the active ingredients can also be dissolved in polyethylene glycol, soybean oil, or the like, and then coated with a gelatin film.

For example, in the manufacture of external preparations for skin, the active ingredients can be added into white petrolatum, beeswax, liquid paraffin, polyethylene glycol or the like, moistened as required, and kneaded to prepare an ointment. For example, those can also be kneaded with binders such as rosin, alkyl acrylate polymer, or the like, and then spread on a non-woven fabric such as polyalkyls to prepared a tape-shape preparation.

For example, those can also be prepared as sustained-release preparations such as implantable sheets or a delivery system encapsulated in microcapsules, which can be prepared with a carrier that can prevent immediate removal from the body. For example, biodegradable and biocompatible polymers such as ethylene-vinyl acetate, polyanhydride, polyglycolic acid, collagen, polyorthoester, polylactic acid or the like can be used. Persons skilled in the art can readily prepare those materials. For example, a suspension of liposome can also be used as the pharmaceutically acceptable carrier. The liposome can be prepared as a lipid composition comprising phosphatidylcholine, cholesterol, and PEG-derivatized phosphatidylethanolamine (PEG-PE), which can be prepared to an appropriate size with a filter of appropriate pore size, through appropriate pore size, and purified by reverse phase evaporation.

In the present application, the dose and number of administration can be properly selected in accordance with conditions such as prophylactic and/or therapeutic purpose of disease progress of the subject to be treated, types of disease, weight and age of patients, or the like. For example, in the case of oral administration, it can be administered once or several times daily, or can be administered every several days. In the case of injections, it can be administered continuously or intermittently.

## Kits

The kit of the present application can comprise the pharmaceutical combination as described in the present application. The pharmaceutical combination can be provided in a form of kit. Various constituents of the pharmaceutical combination can be packaged in different containers, and are mixed before administration or administered separately without mixing.

For example, separate packaging can be for long-term storage without losing the function of the active constituents.

For example, the preparation contained in the kit can each be present in any type of containers in which the ingredients can effectively maintaining the activity for a long term, are not adsorbed by the material of the container, and are not easy to deteriorated. For example, sealed ampules, e.g., made from glass, organic polymers such as polycarbonate, polystyrene and the like, ceramic, metal, or any other suitable materials commonly used for holding an agent, etc. Other suitable examples of containers comprise simple bottles made from materials similar to ampules, or packages lined with foils such as aluminum or alloy foils. Other containers comprise tube, vial, flask, bottle, syringe, or the like. The container has a sterile access port for bottle with a stopper that can be penetrated with an injection needle.

For example, the kit can further comprise a buffer that is packaged in the presence of a neutral, non-reactive gas, such as nitrogen.

For example, the kit can further comprise auxiliary administration devices, e.g., measuring cups, measuring spoons, e.g., syringes suitable for injection, infusion tubes, infusion needles, or the like.

For example, the kit can additionally comprise an instruction for use. The instruction for use of the kit constituted of the pharmaceutical composition can be printed on paper or other materials, and/or can be supplied in an electrically or electromagnetically readable manner such as Floppy discs, CD-ROMs, DVD-ROMs, Zip discs, video tapes, audio tapes, or the like. Detailed instruction for use can be physically attached to the kit, or can be posted on a website designated or notified with email by the manufacturer or distributor.

Without being limited by any theory, the following examples are only for illustrating the pharmaceutical combination and the method and use for treating/preventing cancers of the present application or the like, and are not intended to limit the scope of the invention of the application.

## EXAMPLES

The drugs used in the examples of the present application are listed be in Table 1, all of which are commercially available.

Table 1: List of Drugs

| No. | Drug Name | Item No. | Manufacturer |
|---|---|---|---|
| 1 | Berberine | S9046 | Selleck |
| 2 | MK0752 | T2625 | Targetmol |
| 3 | RO4929097 | S1575 | Selleck |
| 4 | PF-03084014 | S8018 | Selleck |
| 5 | Vismodegib | T2590 | Targetmol |
| 6 | Sonidegib | S2151 | Selleck |
| 7 | Glasdegib | S7160 | Selleck |
| 8 | LGK974 | BD295733 - Bide Pharmatech | NBW |
| 9 | ETC-159 | 24104 | Cayman |
| 11 | Foxy-5 | HY-P1416A | MedChemExpress |
| 12 | Sodium Ascorbate | S105024 | Aladdin |

Where, the drugs numbered 1-12 are used with DMSO as the solvent, and sodium ascorbate is used with PBS as the solvent.

HEK293 cells, HOS human osteosarcoma cells, 143B human osteosarcoma cells, MC38 cells and DB cells in the examples of the present application are all purchased from ATCC.

**Example 1. Inhibitory effect of berberine in combination with sodium ascorbate on osteosarcoma cells**

1.1 Cell line and cell culture

HEK293 cells, HOS human osteosarcoma cells and 143B human osteosarcoma cells were subcultured in an incubator at 37°C, 5% $CO_2$ using a RPMI-1640 medium containing 10% fetal bovine serum (FBS).

1.2 Detection of cell viability of single-drug administration

(1) Cells were plated in a 10 cm culture plate to grow a density of about 90%. The medium was removed, and the adherent cells were washed once with a PBS buffer. 1 mL of trypsin was added, and immediately discarded after mixing well. The cells were placed in the incubator and digested for about 5 min, and then about 10 mL of fresh medium was added. 10 μL was taken for counting.

(2) After counting, the cells were diluted to $1 \times 10^5$ cells/ml. The diluted cells were plated into a 96-well plate with 100 μL per well, i.e., about 10,000 cells per well.

(3) The cells plated in the 96-well plate were cultured for 24 h, and then berberine with different concentration gradients were added thereto, wherein the gradient was set to six concentration gradients of 1 μM, 2 μM, 4 μM, 8 μM, 16 μM, and 32 μM.

(4) After 24h of dosing, 10 μL of CCK-8 solution was added. The cells were incubated in the incubator for 1-2 hours, and then measured at 490 nm using a microplate reader for the absorbance (OD value) of each well. The results were recorded. The relative cell viability was calculated as follows:

$$\text{Cell Viability} = (\text{Absorbance of Test Group} - \text{Absorbance of Blank Group}) / (\text{Absorbance of Control Group} - \text{Absorbance of Blank Group}) \times 100\%,$$

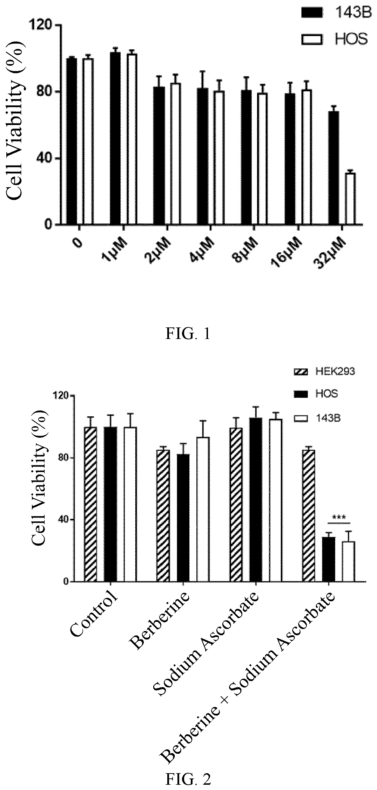

wherein the test group is a cell group to which different concentrations of drugs are added, the control group is a cell group to which only solvent (DMSO) is added, and the blank group is a group to which no cell is added and only solvent (DMSO) is added. The results of cell viability are obtained by the above equation, and the cell inhibition rate of drugs = 100% - the cell viability. The cell viability of the control group obtained according the equation is 100%. The results of cells treated with berberine with the concentration gradient are shown in FIG. 1.

1.3 Cell counts of drug combination administration

(1) Cells were plated in a 10 cm culture plate to grow a density of about 90%. The medium was removed, and the adherent cells were washed once with a PBS buffer. 1 mL of trypsin was added, and immediately discarded after mixing well. The cells were placed in the incubator and digested for about 5 min, and then about 10 mL of fresh medium was added. 10 μL was taken for counting.

(2) 10 μL of the well-mixed cells were taken and mixed into 10 μL of Trypan Blue solution, and counted.

(3) After counting, the cells were diluted to $1 \times 10^5$ cells/ml. The diluted cells were mixed well, and plated into a 24-well plate with 500 μL per well, i.e., about 50,000 cells per well.

(4) The cells plated in the 24-well plate were cultured for about 24 hrs., and then drugs were added. Various test groups comprised a single-drug group of berberine (with a concentration of 20 μM), a group of ascorbic acid (with a concentration of 0.5 mM), a group of berberine (with a concentration of 20 μM) in combination with ascorbic acid (with a concentration of 0.5 mM), a control group that was a cell group to which only the solvent DMSO was added; and a blank group that was a group to which no cell was added and only the solvent (DMSO) was added.

(5) After 24h of dosing, the medium was discarded. About 150 μL of trypsin was added and then discarded. The cells were digested for 5 min.

(6) After digestion, equivalent amounts of PBS were added into each well and mixed uniformly. Then, 10 μL/well of Trypan Blue solution was added and mixed uniformly. 10 μL of the well-mixed solution was taken and counted. In accordance with "Cell viability = Cell Count of Test Group / Cell Count of Control Group $\times$ 100%", the cell viability of various groups was calculated.

1.4 Data processing

Data are all expressed as mean $\pm$ standard deviation (Mean $\pm$ SD), and one-way ANOVA was performed using GraphPad Prism 7.0 software. P<0.05 indicates that the difference is statistically significant. * represents P<0.05, ** represents P<0.01, *** represents P<0.001, and **** represents P<0.0001.

The coefficient of drug in interaction (CDI) was used to evaluate the nature of the interaction between the two drugs. The test groups were divided into the control group, the group of A drug, the group of B drug and the group of AB in combination, and used for detecting the cell viability in A, B and AB groups, respectively.

CDI = the cell viability in group AB / the cell viability in group Ax cell viability in group B. CDI < 1 indicates that the interaction between the two drugs is synergistic; CDI < 0.7 indicates that the interaction between the two drugs is highly synergistic; CDI = 1 indicates the interaction between the two drugs are additive; and CDI > 1 indicates that the interaction between the two drugs is antagonistic.

The results of combination: under the condition of treatment with a single-drug of 20 $\mu$M of berberine, the HOS cells have cell viability of 82%, the 143B cells have cell viability of 94%; under the condition of treatment with 0.5 mM of sodium ascorbate, the HOS cells have cell viability of 106%, the 143B cells have cell viability of 106%; and under the condition of treatment with 20 $\mu$M of berberine in combination with 0.5 mM of sodium ascorbate, the HOS cells have cell viability of 29%, the 143B cells have cell viability of 26%. In accordance with the above-described CDI calculation equation, the CDI value is 0.33 for the HOS cells, and 0.26 for the 143B cells, indicating that they have a synergistic effect therebetween. At the same time, as compared with the HEK293 cells, the combination of berberine with ascorbic acid specifically inhibits the cell viability of the 143B cells and the HOS cells, and has a significantly better inhibitory effect on the 143B cells and the HOS cells than the corresponding single-drug groups (P<0.001). The results are shown in FIG. 2.

## Example 2. Inhibitory effect of Foxy-5 in combination with sodium ascorbate on osteosarcoma cells

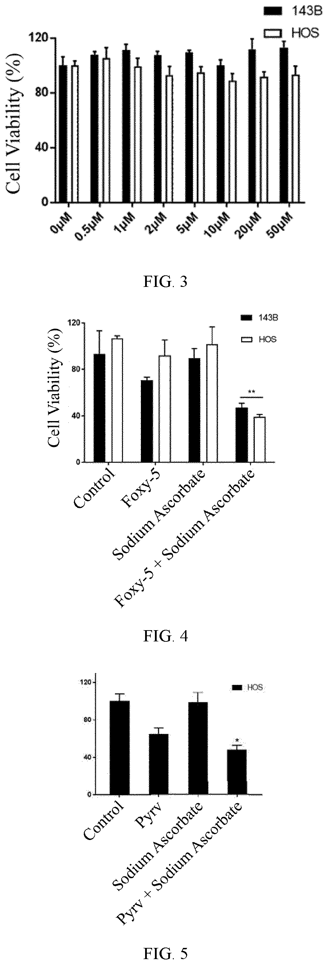

The inhibitory effect of Foxy-5 on the HOS human osteosarcoma cells and the 143B HOS human osteosarcoma cells was detected in accordance with the method of Example 1. The concentration gradients of Foxy-5 and the results of cells treated therewith are shown in FIG. 3.

The results of combination: under the condition of treatment with a single-drug of 10 $\mu$M of Foxy-5, the HOS cells have cell viability of 92%, the 143B cells have cell viability of 71%; under the condition of treatment with 0.5 mM of sodium ascorbate, the HOS cells have cell viability of 103%, the 143B cells have cell viability of 90%; and under the condition of treatment with 10 $\mu$M of Foxy-5 in combination with 0.5 mM of sodium ascorbate, the HOS cells have cell viability of 39%, the 143B cells have cell viability of 47%. In accordance with the above-described CDI calculation equation, the CDI value is 0.41 for the HOS cells, and 0.74 for the 143B cells, indicating that they have a synergistic effect therebetween. At the same time, the inhibitory effect of Foxy-5 in combination with ascorbic acid on both the 143B cells and the HOS cells is significantly better than that of the corresponding single-drug groups (P<0.01). The results are shown in FIG. 4.

## Example 3. Inhibitory effect of pyrvinium in combination with sodium ascorbate on osteosarcoma cells

The inhibitory effect of Pyrvinium on the HOS human osteosarcoma cells was detected in accordance with the method of Example 1.

The results of combination: under the condition of treatment with a single-drug of 0.1 $\mu$M of Pyrvinium, the HOS cells have cell viability of 65%; under the condition of treatment with 0.5 mM of sodium ascorbate, the HOS cells have cell viability of 99%; and under the condition of treatment with 0.1 $\mu$M of Pyrvinium in combination with 0.5 mM of sodium ascorbate, the HOS cells have cell viability of 49%. In accordance with the above-described CDI calculation equation, the CDI value is 0.76 for the HOS cells, indicating that they have a synergistic effect therebetween. At the same time, the inhibitory effect of Pyrvinium in combination with ascorbic acid on the HOS cells is significantly better than that of the corresponding single-drug groups (P<0.05). The results are shown in FIG. 5.

## Example 4. Inhibitory effect of niclosamide in combination with sodium ascorbate on osteosarcoma cells

The inhibitory effect of Niclosamide on the HOS human osteosarcoma cells and the 143B HOS human osteosarcoma cells was detected in accordance with the method of Example 1.

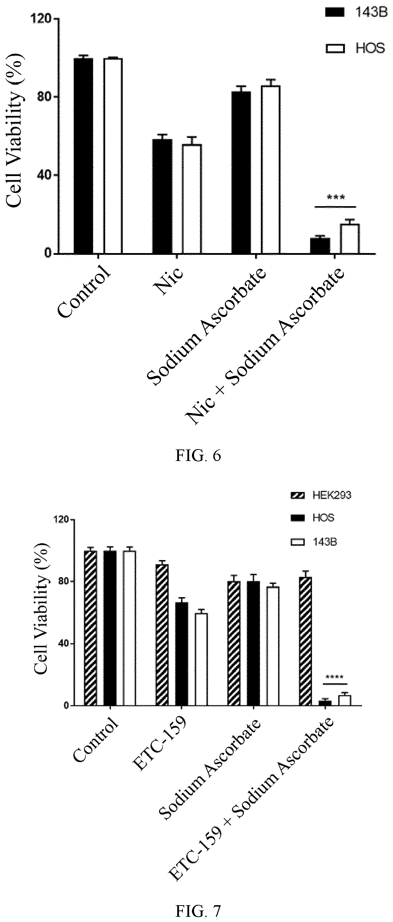

The results of combination: under the condition of treatment with a single-drug of 50 $\mu$M of Niclosamide, the HOS cells have cell viability of 56%, the 143B cells have cell viability of 59%; under the condition of treatment with 0.5 mM of sodium ascorbate, the HOS cells have cell viability of 86%, the 143B cells have cell viability of 83%; and under the condition of treatment with 50 $\mu$M of Niclosamide in combination with 0.5 mM of sodium ascorbate, the HOS cells have cell viability of 15%, the 143B cells have cell viability of 8%. In accordance with the above-described CDI calculation equation, the CDI value is 0.31 for the HOS cells, and 0.16 for the 143B cells, indicating that they have a synergistic effect therebetween. At the same time, the inhibitory effect of Niclosamide in combination with ascorbic acid on both the 143B cells and the HOS cells is significantly better than that of the corresponding single-drug groups (P<0.001). The results are shown in FIG. 6.

**Example 5. Inhibitory effect of ETC-159 in combination with sodium ascorbate on osteosarcoma cells**

The inhibitory effect of ETC-159 on the HOS human osteosarcoma cells and the 143B HOS human osteosarcoma cells was detected in accordance with the method of Example 1.
The results of combination: under the condition of treatment with a single-drug of 20 $\mu$M of ETC-159, the HOS cells have cell viability of 67%, the 143B cells have cell viability of 60%; under the condition of treatment with 0.5 mM of sodium ascorbate, the HOS cells have cell viability of 80%, the 143B cells have cell viability of 77%; and under the condition of treatment with 20 $\mu$M of ETC-159 in combination with 0.5 mM of sodium ascorbate, the HOS cells have cell viability of 3%, the 143B cells have cell viability of 7%. In accordance with the above-described CDI calculation equation, the CDI value is 0.06 for the HOS cells, and 0.15 for the 143B cells, indicating that they have a synergistic effect therebetween. At the same time, as compared with the HEK293 cells, the combination of ETC-159 with ascorbic acid specifically inhibits the cell viability of the 143B cells and the HOS cells, and has a significantly better inhibitory effect on both the 143B cells and the HOS cells than the corresponding single-drug groups (P<0.001). The results are shown in FIG. 7.

**Example 6. Inhibitory effect of LGK974 in combination with sodium ascorbate on osteosarcoma cells**

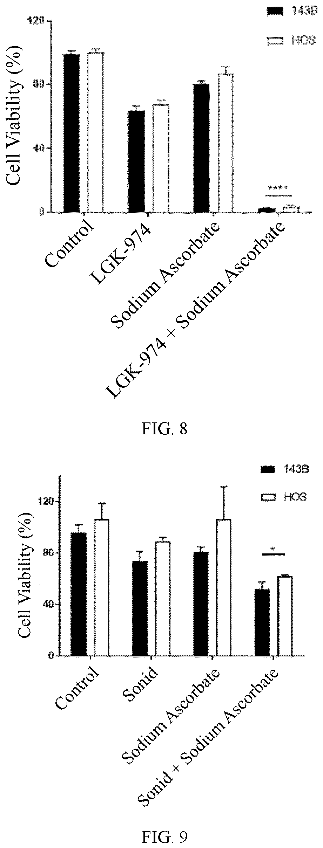

The inhibitory effect of LGK974 on the HOS human osteosarcoma cells and the 143B HOS human osteosarcoma cells was detected in accordance with the method of Example 1.
The results of combination: under the condition of treatment with a single-drug of 20 $\mu$M of LGK974, the HOS cells have cell viability of 67%, the 143B cells have cell viability of 64%; under the condition of treatment with 0.5 mM of sodium ascorbate, the HOS cells have cell viability of 87%, the 143B cells have cell viability of 80%; and under the condition of treatment with 20 $\mu$M of LGK974 in combination with 0.5 mM of sodium ascorbate, the HOS cells have cell viability of 4%, the 143B cells have cell viability of 2%. In accordance with the above-described CDI calculation equation, the CDI value is 0.07 for the HOS cells, and 0.04 for the 143B cells, indicating that they have a synergistic effect therebetween. At the same time, the inhibitory effect of LGK974 in combination with ascorbic acid on both the 143B cells and the HOS cells is significantly better than that of the corresponding single-drug groups (P<0.001). The results are shown in FIG. 8.

**Example 7. Inhibitory effect of Sonidegib in combination with sodium ascorbate on osteosarcoma cells**

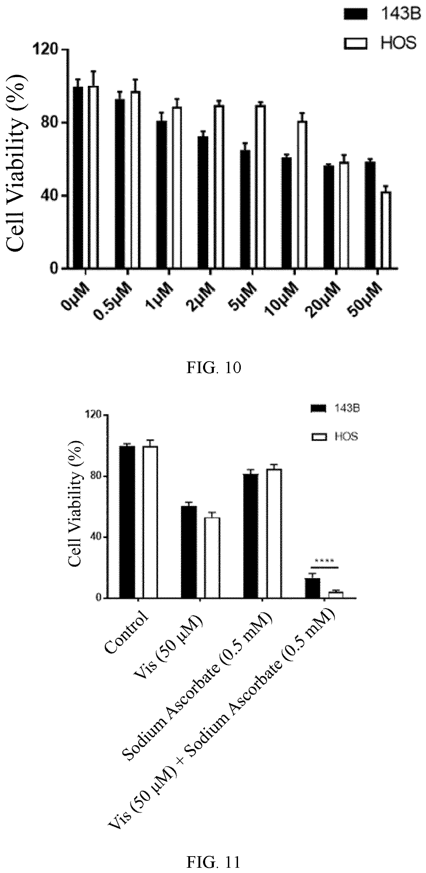

The inhibitory effect of Sonidegib on the HOS human osteosarcoma cells and the 143B HOS human osteosarcoma cells was detected in accordance with the method of Example 1. The concentration gradients of Sonidegib and the results of cells treated therewith are shown in FIG. 9.
The results of combination: under the condition of treatment with a single-drug of 10 $\mu$M of Sonidegib, the HOS cells have cell viability of 89%, the 143B cells have cell viability of 74%; under the condition of treatment with 0.5 mM of sodium ascorbate, the HOS cells have cell viability of 106%, the 143B cells have cell viability of 81%; and under the condition of treatment with 10 $\mu$M of Sonidegib in combination with 0.5 mM of sodium ascorbate, the HOS cells have cell viability of 62%, the 143B cells have cell viability of 52%. In accordance with the above-described CDI calculation equation, the CDI value is 0.66 for the HOS cells, and 0.87 for the 143B cells, indicating that they have a synergistic effect therebetween. At the same time, the inhibitory effect of Sonidegib in combination with ascorbic acid on both the 143B cells and the HOS cells is significantly better than that of the corresponding single-drug groups (P<0.05). The results are shown in FIG. 10.

**Example 8. Inhibitory effect of Vismodegib in combination with sodium ascorbate on osteosarcoma cells**

The inhibitory effect of Vismodegib on the HOS human osteosarcoma cells and the 143B HOS human osteosarcoma cells was detected in accordance with the method of Example 1.
The results of combination: under the condition of treatment with a single-drug of 50 $\mu$M of Vismodegib, the HOS cells have cell viability of 53%, the 143B cells have cell viability of 60%; under the condition of treatment with 0.5 mM of sodium ascorbate, the HOS cells have cell viability of 106%, the 143B cells have cell viability of 82%; and under the condition of treatment with 50 $\mu$M of Vismodegib in combination with 0.5 mM of sodium ascorbate, the HOS cells have cell viability of 84%, the 143B cells have cell viability of 13%. In accordance with the above-described CDI calculation equation, the CDI value is 0.09 for the HOS cells, and 0.26 for the 143B cells, indicating that they have a synergistic effect therebetween. At the same time, the inhibitory effect of Vismodegib in combination with ascorbic acid on both the 143B cells and the HOS cells is significantly better than that of the corresponding single-drug groups (P<0.001). The results are shown in FIG. 11.

**Example 9. Inhibitory effect of PF-03084014 in combination with sodium ascorbate on osteosarcoma cells**

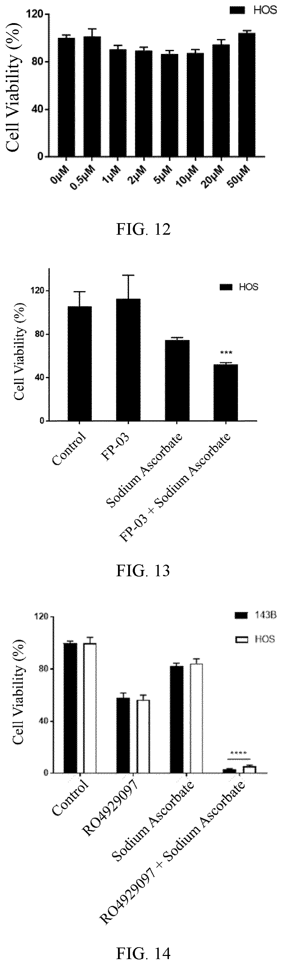

The inhibitory effect of PF-03084014 on the HOS human osteosarcoma cells was detected in accordance with the method of Example 1. The concentration gradients of PF-03084014 and the results of cells treated therewith are shown in FIG. 12.

The results of combination: under the condition of treatment with a single-drug of 10 $\mu$M of PF-03084014, the HOS cells have cell viability of 113%; under the condition of treatment with 0.5 mM of sodium ascorbate, the HOS cells have cell viability of 75%; and under the condition of treatment with 10 $\mu$M of PF-03084014 in combination with 0.5 mM of sodium ascorbate, the HOS cells have cell viability of 52%. In accordance with the above-described CDI calculation equation, the CDI value is 0.61 for the HOS cells, indicating that they have a synergistic effect therebetween. At the same time, the inhibitory effect of PF-03084014 in combination with ascorbic acid on the HOS cells is significantly better than that of the corresponding single-drug groups (P<0.001). The results are shown in FIG. 13.

**Example 10. Inhibitory effect of RO4929097 in combination with sodium ascorbate on osteosarcoma cells**

The inhibitory effect of RO4929097 on the HOS human osteosarcoma cells and the 143B HOS human osteosarcoma cells was detected in accordance with the method of Example 1.

The results of combination: under the condition of treatment with a single-drug of 10 $\mu$M of RO4929097, the HOS cells have cell viability of 56%, the 143B cells have cell viability of 58%; under the condition of treatment with 0.5 mM of sodium ascorbate, the HOS cells have cell viability of 84%, the 143B cells have cell viability of 82%; and under the condition of treatment with 10 $\mu$M of RO4929097 in combination with 0.5 mM of sodium ascorbate, the HOS cells have cell viability of 5%, the 143B cells have cell viability of 3%. In accordance with the above-described CDI calculation equation, the CDI value is 0.11 for the HOS cells, and 0.06 for the 143B cells, indicating that they have a synergistic effect therebetween. At the same time, the inhibitory effect of RO4929097 in combination with ascorbic acid on both the 143B cells and the HOS cells is significantly better than that of the corresponding single-drug groups (P<0.001). The results are shown in FIG. 14.

**Example 11. Inhibitory effect of MK0752 in combination with sodium ascorbate on osteosarcoma cells**

The inhibitory effect of MK0752 on the HOS human osteosarcoma cells and the 143B HOS human osteosarcoma cells was detected in accordance with the method of Example 1.

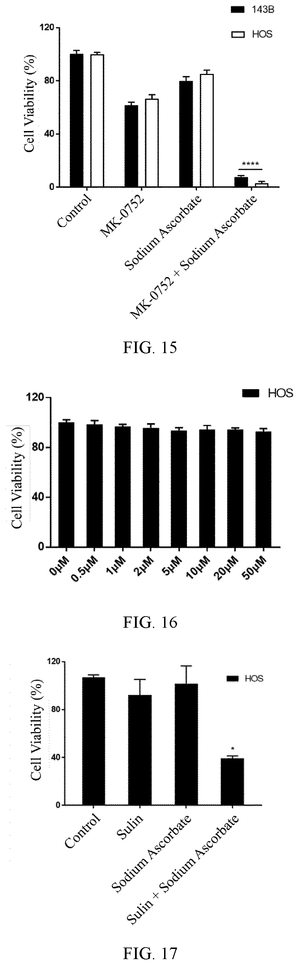

The results of combination: under the condition of treatment with a single-drug of 20 $\mu$M of MK0752, the HOS cells have cell viability of 67%, the 143B cells have cell viability of 62%; under the condition of treatment with 0.5 mM of sodium ascorbate, the HOS cells have cell viability of 85%, the 143B cells have cell viability of 80%; and under the condition of treatment with 20 $\mu$M of MK0752 in combination with 0.5 mM of sodium ascorbate, the HOS cells have cell viability of 3%, the 143B cells have cell viability of 8%. In accordance with the above-described CDI calculation equation, the CDI value is 0.05 for the HOS cells, and 0.16 for the 143B cells, indicating that they have a synergistic effect therebetween. At the same time, the inhibitory effect of MK0752 in combination with ascorbic acid on both the 143B cells and the HOS cells is significantly better than that of the corresponding single-drug groups (P<0.001). The results are shown in FIG. 15.

**Example 12. Inhibitory effect of Sulindac in combination with sodium ascorbate on osteosarcoma cells**

The inhibitory effect of Sulindac on the HOS human osteosarcoma cells was detected in accordance with the method of Example 1. The concentration gradients of Sulindac and the results of cells treated therewith are shown in FIG. 16.

The results of combination: under the condition of treatment with a single-drug of 50 $\mu$M of Sulindac, the HOS cells have cell viability of 92%; under the condition of treatment with 0.5 mM of sodium ascorbate, the HOS cells have cell viability of 102%; and under the condition of treatment with 50 $\mu$M of Sulindac in combination with 0.5 mM of sodium ascorbate, the HOS cells have cell viability of 39%. In accordance with the above-described CDI calculation equation, the CDI value is 0.42 for the HOS cells, indicating that they have a synergistic effect therebetween. At the same time, the inhibitory effect of Sulindac in combination with ascorbic acid on the HOS cells is significantly better than that of the corresponding single-drug groups (P<0.05). The results are shown in FIG. 17.

**Example 13. Inhibitory effects of various inhibitors in combination with sodium ascorbate on colon cancer cells**

The inhibitory effects of berberine, Foxy-5, Pyrvinium, Niclosamide, ETC-159, LGK974, Sonidigib, Vismodegib, PF-03084014, RO4929097, or MK0752 on the colon cancer cells were detected in accordance with the method of Example 1.

For example, as for MC38 colon cancer cells, the control group involves adding the solvent DMSO alone, the Nic group involves adding 1 $\mu$M of Niclosamide, the Vc group involves adding 0.5 mM of ascorbic acid, the Nic + Vc group involves adding 1 $\mu$M of Niclosamide and 0.5 mM of ascorbic acid.

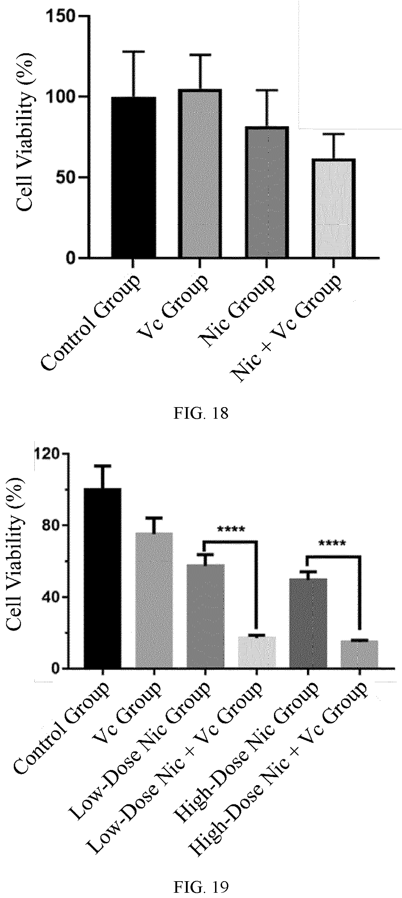

Fig. 18 shows that, as compared with the cell viability of the control group, the MC38 cells have cell viability of 82% under the condition of treatment with 1 $\mu$M of Niclosamide; 105% under the condition of treatment with 0.5 mM of ascorbic acid; and 62% under the condition of treatment with 1 $\mu$M of Niclosamide and 0.5 mM of ascorbic acid. The coefficient of drug interaction (CDI) for the MC38 cells obtained in accordance with the CDI calculation equation in Example 1 is 0.72, indicating that they have significant synergistic effect therebetween. At the same time, the Niclosamide in combination with ascorbic acid has a better inhibitor effect on the MC38 cells than the corresponding single-drug groups.

The results show that the respective combination of the aforesaid drugs (e.g., the Wnt signaling pathway inhibitor) with sodium ascorbate has a significantly better inhibitory effect on colon cancer cells than the corresponding single-drug groups, and they have synergistic effect therebetween.

**Example 14. Inhibitory effects of various inhibitors in combination with sodium ascorbate on lymphoma cells**

The inhibitory effects of berberine, Foxy-5, Pyrvinium, Niclosamide, ETC-159, LGK974, Sonidigib, Vismodegib, PF-03084014, RO4929097, MK0752, Sulindac on the lymphoma cells were detected in accordance with the method of Example 1.

For example, as for the DB lymphoma cells, the control group involves adding the solvent DMSO alone, the Vc group involves adding 2 mM of ascorbic acid, the low-dose Nic group involves adding 1 $\mu$M of Niclosamide, the low-dose Nic + Vc group involves adding 1 $\mu$M of Niclosamide and 2 mM of ascorbic acid, the high-dose Nic group involves adding 2 $\mu$M of Niclosamide, and the high-dose Nic + Vc group involves adding 2 $\mu$M of Niclosamide and 2 mM of ascorbic acid.

Fig. 19 shows that, as compared with the cell viability of the control group, the DB cells have cell viability of 76% under the condition of treatment with 2 mM of ascorbic acid; 57% under the condition of treatment with 1 $\mu$M of Niclosamide; 17% under the condition of treatment with 1 $\mu$M of Niclosamide and 2 mM of ascorbic acid; 52% under the condition of treatment with 2 $\mu$M of Niclosamide; and 15% under the condition of treatment of 2 $\mu$M of Niclosamide and 2 mM of ascorbic acid. The coefficient of drug interaction for DB cells is obtained in accordance with the CDI calculation equation in Example 1, the CDI value for the combination of the low-dose Niclosamide with ascorbic acid is 0.39, and the CDI value for the combination of the high-dose Niclosamide with ascorbic acid is 0.38, indicating that they have significant synergistic effect therebetween. At the same time, the inhibitory effect of both the low- and the high-dose Niclosamide in combination with ascorbic acid on the DB cells is significantly better than that of the corresponding single-drug groups (P<0.0001).

The results show that the respective combination of the aforesaid drugs (e.g., the Wnt signaling pathway inhibitor) with sodium ascorbate has a significantly better inhibitory effect on lymphoma cells than the corresponding single-drug groups, and they have synergistic effect therebetween.

The aforesaid detailed description is provided in an illustrative and exemplary manner, and are not intended to limit the scope of the appended claims. Various modifications of embodiments currently listed in the present application are apparent for persons skilled in the art, and encompassed within the scope of the appended claims and their equivalences.

**Claims**

1. A pharmaceutical combination, comprising:

   a) a prophylactically and/or therapeutically effective amount of ascorbic acid or a derivative thereof; and
   b) a prophylactically and/or therapeutically effective amount of a second therapeutic agent selected from the group consisting of: a Notch signaling pathway inhibitor, an HH signaling pathway inhibitor, a Porcupine inhibitor, an RXR$\alpha$ inhibitor, Niclosamide, a CK1a inhibitor, and a Frizzled receptor inhibitor.

2. The pharmaceutical combination according to claim 1, wherein the RXRα inhibitor comprises berberine or a pharmaceutically acceptable salt thereof.

3. The pharmaceutical combination according to any one of claims 1-2, wherein the Notch signaling pathway inhibitor comprises a γ-secretase inhibitor.

4. The pharmaceutical combination according to claim 3, wherein the γ-secretase inhibitor comprises MK0752, RO4929097, and/or PF-03084014.

5. The pharmaceutical combination according to any one of claims 1-4, wherein the HH signaling pathway inhibitor comprises an SMO inhibitor.

6. The pharmaceutical combination according to claim 5, wherein the SMO inhibitor comprises Vismodegib, Sonidegib, and/or Glasdegib.

7. The pharmaceutical combination according to any one of claims 1-6, wherein the Porcupine inhibitor comprises LGK974 and/or ETC-159.

8. The pharmaceutical combination according to any one of claims 1-7, wherein the CK1a inhibitor comprises Pyrvinium.

9. The pharmaceutical combination according to any one of claims 1-8, wherein the Frizzled receptor inhibitor comprises Foxy-5.

10. The pharmaceutical combination according to any one of claims 1-9, wherein the derivative of ascorbic acid comprises a pharmaceutically acceptable ascorbate.

11. The pharmaceutical combination according to claim 10, wherein the pharmaceutically acceptable salt of ascorbic acid comprises sodium ascorbate.

12. The pharmaceutical combination according to any one of claims 1-11, wherein a ratio of the effective amount of the second therapeutic agent to the effective amount of the ascorbic acid or a derivative thereof is about 1:10 to about 1:5000.

13. The pharmaceutical combination according to any one of claims 1-12, wherein a mass ratio of the second therapeutic agent to the ascorbic acid or a derivative thereof is about 1:10 to about 1:5000.

14. The pharmaceutical combination according to any one of claims 1-13, wherein the second therapeutic agent and the ascorbic acid or a derivative thereof are each present in different containers.

15. The pharmaceutical combination according to any one of claims 1-14, wherein the pharmaceutical combination comprises a first preparation and a second preparation, wherein the first preparation comprises the ascorbic acid or a derivative thereof and a pharmaceutically acceptable first carrier, and the second preparation comprises the second therapeutic agent and a pharmaceutically acceptable second carrier.

16. The pharmaceutical combination according to any one of claims 1-13, which comprises a pharmaceutical composition comprising the second therapeutic agent and the ascorbic acid or a derivative thereof.

17. The pharmaceutical combination according to claim 16, wherein the second therapeutic agent has a content of about 5% to about 20% (w/w) in the pharmaceutical composition.

18. The pharmaceutical combination according to any one of claims 16-17, wherein the ascorbic acid or a derivative thereof has a content of about 80% to about 95% (w/w) in the pharmaceutical composition.

19. A kit comprising the pharmaceutical combination according to any one of claims 1-18.

20. Use of a combination of ascorbic acid or a derivative thereof with a second therapeutic agent in the manufacture of a drug for preventing and/or treating tumors, wherein the second therapeutic agent is selected from the group consisting of: a Notch signaling pathway inhibitor, an HH signaling pathway inhibitor, an RXRα inhibitor, and a Wnt signaling pathway inhibitor.

21. The use according to claim 20, wherein the Wnt signaling pathway inhibitor comprises a Porcupine inhibitor, a Dvl-2 inhibitor, a CK1a inhibitor, and/or a Frizzled receptor inhibitor.

22. The use according to claim 21, wherein the Porcupine inhibitor comprises LGK974, and/or ETC-159.

23. The use according to any one of claims 21-22, wherein the Dvl-2 inhibitor comprises Niclosamide and/or Sulindac.

24. The use according to any one of claims 21-23, wherein the CK1a inhibitor comprises Pyrvinium.

25. The use according to any one of claims 21-24, wherein the Frizzled receptor inhibitor comprises Foxy-5.

26. The use according to any one of claims 20-25, wherein the RXR$\alpha$ inhibitor comprises berberine or a pharmaceutically acceptable salt thereof.

27. The use according to any one of claims 20-26, wherein the Notch signaling pathway inhibitor comprises a $\gamma$-secretase inhibitor.

28. The use according to claim 27, wherein the $\gamma$-secretase inhibitor comprises MK0752, RO4929097, and/or PF-03084014.

29. The use according to any one of claims 20-28, wherein the HH signaling pathway inhibitor comprises an SMO inhibitor.

30. The use according to claim 29, wherein the SMO inhibitor comprises Vismodegib, Sonidegib, and/or Glasdegib.

31. The use according to any one of claims 20-30, wherein the tumors comprise solid tumors and non-solid tumors.

32. The use according to claim 31, wherein the solid tumors comprise osteosarcoma and/or colon cancer.

33. The use according to any one of claims 31-32, wherein the non-solid tumors comprise lymphoma.

34. The use according to any one of claims 20-33, wherein the derivative of ascorbic acid comprises a pharmaceutically acceptable ascorbate.

35. The use according to claim 34, wherein the pharmaceutically acceptable ascorbate comprises sodium ascorbate.

36. The use according to any one of claims 20-35, wherein the second therapeutic agent and the ascorbic acid or a derivative thereof are formulated to be simultaneously administered to a subj ect.

37. The use according to any one of claims 20-35, wherein the second therapeutic agent and the ascorbic acid or a derivative thereof are formulated to be separately administered to a subj ect.

38. The use according to any one of claims 20-37, wherein the drug is formulated so that the second therapeutic agent is administered at a dose of about 1 mg/kg to about 100 mg/kg.

39. The use according to any one of claims 20-38, wherein the drug is formulated so that the ascorbic acid or a derivative thereof is administered at a dose of about 0.05 g/kg to about 2.5 g/kg.

40. The use according to any one of claims 20-39, wherein the drug is formulated so that the second therapeutic agent and the ascorbic acid or a derivative thereof are administered at a mass ratio of about 1:5 to about 1:5000.

41. The use according to any one of claims 20-40, wherein the drug is formulated so that the second therapeutic agent and the ascorbic acid or a derivative thereof are administered at an effective amount ratio of about 1:5 to about 1:5000.

42. A method for preventing and/or treating tumors, comprising administering to a subject in need thereof:

a) ascorbic acid or a derivative thereof; and

b) a second therapeutic agent, wherein the second therapeutic agent is selected from the group consisting of: a Notch signaling pathway inhibitor, an HH signaling pathway inhibitor, an RXRα inhibitor, and a Wnt signaling pathway inhibitor.

43. The method according to claim 42, wherein the Wnt signaling pathway inhibitor comprises a Porcupine inhibitor, a Dvl-2 inhibitor, a CK1a inhibitor, and/or a Frizzled receptor inhibitor.

44. The method according to claim 43, wherein the Porcupine inhibitor comprises LGK974 and/or ETC-159.

45. The method according to any one of claims 43-44, wherein the Dvl-2 inhibitor comprises Niclosamide and/or Sulindac.

46. The method according to any one of claims 43-45, wherein the CK1a inhibitor comprises Pyrvinium.

47. The method according to any one of claims 43-46, wherein the Frizzled receptor inhibitor comprises Foxy-5.

48. The method according to any one of claims 43-47, wherein the RXRα inhibitor comprises berberine or a pharmaceutically acceptable salt thereof.

49. The method according to any one of claims 42-48, wherein the Notch signaling pathway inhibitor comprises a γ-secretase inhibitor.

50. The method according to claim 49, wherein the γ-secretase inhibitor comprises MK0752, RO4929097, and/or PF-03084014.

51. The method according to any one of claims 42-50, wherein the HH signaling pathway inhibitor comprises an SMO inhibitor.

52. The method according to claim 51, wherein the SMO inhibitor comprises Vismodegib, Sonidegib, and/or Glasdegib.

53. The method according to claim 42-52, wherein the tumors comprise solid tumors and non-solid tumors.

54. The method according to claim 53, wherein the solid tumors comprise osteosarcoma and/or colon cancer.

55. The method according to any one of claims 53-54, wherein the non-solid tumors comprise lymphoma.

56. The method according to any one of claims 42-55, wherein the derivative of ascorbic acid comprises a pharmaceutically acceptable ascorbate.

57. The method according to claim 56, wherein the pharmaceutically acceptable ascorbate comprises sodium ascorbate.

58. The method according to any one of claims 42-57, wherein the second therapeutic agent and the ascorbic acid or a derivative thereof are formulated to be simultaneously administered to a subject.

59. The method according to any one of claims 42-57, wherein the second therapeutic agent and the ascorbic acid or a derivative thereof are formulated to be separately administered to a subject.

60. The method according to any one of claims 42-59, wherein the drug is formulated so that the second therapeutic agent is administered at a dose of about 1 mg/kg to about 100 mg/kg.

61. The method according to any one of claims 42-60, wherein the drug is formulated so that the ascorbic acid or a derivative thereof is administered at a dose of about 0.05 g/kg to about 2.5 g/kg.

62. The method according to any one of claims 42-61, wherein the drug is formulated so that the second therapeutic agent and the ascorbic acid or a derivative thereof are administered at a mass ratio of about 1:5 to about 1:5000.

63. The method according to any one of claims 42-62, wherein the drug is formulated so that the second therapeutic agent and the ascorbic acid or a derivative thereof are administered at an effective amount ratio of about 1:5 to about 1:5000.

64. A method for monitoring a response to a drug in a subject, comprising:

a) administering to the subject ascorbic acid or a derivative thereof and a second therapeutic agent, wherein the second therapeutic agent is selected from the group consisting of: a Notch signaling pathway inhibitor, an HH signaling pathway inhibitor, an RXRα inhibitor, and a Wnt signaling pathway inhibitor; and
b) detecting a change in one or more of the level and/or activity of blood alkaline phosphatase, the level and/or activity of lactic dehydrogenase, the expression level of CADM1 gene, the expression level of CD44 gene, the expression level of livin gene, the expression level of HIF-1α gene, the expression level of ET-1 gene, the expression level of IGF-1R gene, the expression level of STAT3 gene, the expression level of CEA gene, the expression level of CA199 gene, the expression level of CA125 gene, the expression level of AFP gene, the expression level of CA724 gene, and the expression level of β-HCG gene in the subject after the administration of a).

65. The method according to claim 64, wherein the Wnt signaling pathway inhibitor comprises a Porcupine inhibitor, a Dvl-2 inhibitor, a CK1a inhibitor, and/or a Frizzled receptor inhibitor.

66. The method according to claim 65, wherein the Porcupine inhibitor comprises LGK974 and/or ETC-159.

67. The method according to any one of claims 65-66, wherein the Dvl-2 inhibitor comprises Niclosamide and/or Sulindac.

68. The method according to any one of claims 65-67, wherein the CK1a inhibitor comprises Pyrvinium.

69. The method according to any one of claims 65-68, wherein the Frizzled receptor inhibitor comprises Foxy-5.

70. The method according to any one of claims 64-69, wherein the RXRα inhibitor comprises berberine or a pharmaceutically acceptable salt thereof.

71. The method according to any one of claims 64-70, wherein the Notch signaling pathway inhibitor comprises a γ-secretase inhibitor.

72. The method according to claim 71, wherein the γ-secretase inhibitor comprises MK0752, RO4929097, and/or PF-03084014.

73. The method according to any one of claims 64-72, wherein the HH signaling pathway inhibitor comprises an SMO inhibitor.

74. The method according to claim 73, wherein the SMO inhibitor comprises Vismodegib, Sonidegib, and/or Glasdegib.

75. The method according to claim 64-74, wherein the tumors comprise solid tumors and non-solid tumors.

76. The method according to claim 75, wherein the solid tumors comprise osteosarcoma and/or colon cancer.

77. The method according to any one of claims 75-76, wherein the non-solid tumors comprise lymphoma.

78. The method according to any one of claims 64-77, wherein the derivative of ascorbic acid comprises a pharmaceutically acceptable ascorbate.

79. The method according to claim 78, wherein the pharmaceutically acceptable ascorbate comprises sodium ascorbate.

80. The method according to any one of claims 64-79, wherein the second therapeutic agent and the ascorbic acid or a derivative thereof are formulated to be simultaneously administered to a subject.

81. The method according to any one of claims 64-79, wherein the second therapeutic agent and the ascorbic acid or a derivative thereof are formulated to be separately administered to a subject.

82. The method according to any one of claims 64-81, wherein the drug is formulated so that the second therapeutic agent is administered at a dose of about 1 mg/kg to about 100 mg/kg.

83. The method according to any one of claims 64-82, wherein the drug is formulated so that the ascorbic acid or a derivative thereof is administered at a dose of about 0.05 g/kg to about 2.5 g/kg.

84. The method according to any one of claims 64-83, wherein the drug is formulated so that the second therapeutic agent and the ascorbic acid or a derivative thereof are administered at a mass ratio of 1:5 to about 1:5000.

85. The method according to any one of claims 64-84, wherein the drug is formulated so that the second therapeutic agent and the ascorbic acid or a derivative thereof are administered at an effective amount ratio of 1:5 to about 1:5000.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

## INTERNATIONAL SEARCH REPORT

| International application No. |
| --- |
| **PCT/CN2021/084008** |

**A.    CLASSIFICATION OF SUBJECT MATTER**

A61K 31/375(2006.01)i;  A61K 45/06(2006.01)i;  A61P 35/00(2006.01)i;  A61P 35/02(2006.01)i;  A61P 35/04(2006.01)i

According to International Patent Classification (IPC) or to both national classification and IPC

**B.    FIELDS SEARCHED**

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

DWPI, SIPOABS, CNABS, CNTXT, TWTXT, EPTXT, WOTXT, USTXT, CNKI(CN), MEDLINE, CAPLUS: 维生素C, 抗坏血酸, Notch, 刻缺蛋白, 伽马分泌酶抑制剂, 癌, 瘤, MK0752, RO4929097, PF03084014, vitamin c, ascorbic acid, gamma secretase inhibit+, cancer, tumor, carcinoma, neoplasm,

**C.    DOCUMENTS CONSIDERED TO BE RELEVANT**

| Category* | Citation of document, with indication, where appropriate, of the relevant passages | Relevant to claim No. |
| --- | --- | --- |
| Y | CN 104136027 A (ECOLE POLYTECHNIQUE FEDERALE DE LAUSANNE (EPFL)) 05 November 2014 (2014-11-05)<br>the abstract, description, paragraph [0029] | 1, 3, 4, 10-19, 20, 27, 31-41 |
| Y | WO 2011060051 A1 (UNIVERSITY OF MASSACHUSETTS) 19 May 2011 (2011-05-19)<br>the abstract, and description, pages 3 and 10 | 1, 3, 4, 10-19, 20, 27, 31-41 |
| Y | 陈虎 等 (CHEN, Hu et al.). "γ-分泌酶抑制剂对骨肉瘤细胞的作用 (Non-official translation: Effects of γ-Secretase Inhibitors on Osteosarcoma Cells)"<br>*中华实验外科杂志 (Chinese Journal of Experimental Surgery)*,<br>Vol. 31, No. 4, 30 April 2014 (2014-04-30),<br>pp. 832-834, especially the abstract | 1, 3, 4, 10-19, 20, 27, 31-41 |
| Y | Rebecca A. Previsl et al. "Molecular Pathways: Translational and Therapeutic Implications of the Notch Signaling Pathway in Cancer"<br>*Clinical Cancer Research*, Vol. 21, No. 5, 01 March 2015 (2015-03-01),<br>pages 955-961, especially the abstract, and pages 4 and 5, GSIs part | 1, 3, 4, 10-19, 20, 27, 31-41 |

☑ Further documents are listed in the continuation of Box C.          ☑ See patent family annex.

| | | |
| --- | --- | --- |
| * | Special categories of cited documents: | |
| "A" | document defining the general state of the art which is not considered to be of particular relevance | "T"  later document published after the international filing date or priority date and not in conflict with the application but cited to understand the principle or theory underlying the invention |
| "E" | earlier application or patent but published on or after the international filing date | "X"  document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&"  document member of the same patent family |

| Date of the actual completion of the international search | Date of mailing of the international search report |
| --- | --- |
| **17 June 2021** | **12 July 2021** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| | International application No. |
|---|---|
| | **PCT/CN2021/084008** |

**C. DOCUMENTS CONSIDERED TO BE RELEVANT**

| Category* | Citation of document, with indication, where appropriate, of the relevant passages | Relevant to claim No. |
|---|---|---|
| X | Rebecca A. Previsl et al. "Molecular Pathways: Translational and Therapeutic Implications of the Notch Signaling Pathway in Cancer" <br> *Clinical Cancer Research*, Vol. 21, No. 5, 01 March 2015 (2015-03-01), <br> pages 955-961, especially the abstract, and pages 4 and 5, GSIs part | 1, 3, 4, 10-11, 16, 17, 19 |
| Y | 张科生 (ZHANG, Kesheng). "大剂量抗坏血酸静脉滴注治疗恶性肿瘤的进展 (Progress of Cancer Treatment by Intravenous High-dose Ascorbic Acid)" <br> 医学与哲学 *(Medicine & Philosophy)*, Vol. 34, No. 6B, 30 June 2013 (2013-06-30), <br> pages 69-72 and 76, especially page 70 | 1, 3, 4, 10-19, 20, 27, 31-41 |
| A | CN 105658787 A (UNIVERSITY COLLEGE CARDIFF CONSULTANTS LTD.) 08 June 2016 (2016-06-08) <br> claims 22 and 12 | 1, 3, 4, 10-19, 20, 27, 28, 31-41 |
| A | CN 107075471 A (AGENCY FOR SCIENCE, TECHNOLOGY AND RESEARCH) 18 August 2017 (2017-08-18) <br> claim 56, and description, paragraphs [0147], [0148] and [0241] | 1, 3, 4, 10-19, 20, 27, 28, 31-41 |
| A | CN 110923191 A (INSTITUTE OF ZOOLOGY, CHINESE ACADEMY OF SCIENCES) 27 March 2020 (2020-03-27) <br> entire document, especially claims 1-3 | 1, 3, 4, 10-19, 20, 27, 28, 31-41 |

Form PCT/ISA/210 (second sheet) (January 2015)

**INTERNATIONAL SEARCH REPORT**

International application No.

**PCT/CN2021/084008**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **42-85**
   because they relate to subject matter not required to be searched by this Authority, namely:

   [1]  The claims relate to a method of treatment of diseases, and therefore do not warrant an international search according to the criteria set out in PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |

This International Searching Authority found multiple inventions in this international application, as follows:

[1]  This application comprises the following seven groups of claims:

[2]  Group 1: claims 1, 3, 4, 10-19, 20, 27, 28 and 31-41: pharmaceutical compositions and their use in the preparation of drugs for the prevention and/or treatment of tumors comprising: ascorbic acid or derivatives thereof, and a second therapeutic agent Notch signaling pathway inhibitor.

[3]  Group 2: claims 1, 2, 10-19, 20, 26 and 31-41: pharmaceutical compositions and their use in the preparation of drugs for the prevention and/or treatment of tumors comprising: ascorbic acid or derivatives thereof, and a second therapeutic agent RXRα inhibitor.

[4]  Group 3: claims 1, 5-6, 10-19, 20, 29, 30 and 31-41: pharmaceutical compositions and their use in the preparation of drugs for the prevention and/or treatment of tumors comprising: ascorbic acid or derivatives thereof, and a second therapeutic agent HH signaling pathway inhibitor.

[5]  Group 4: claims 1, 7, 10-19, 20-22 and 31-41: pharmaceutical compositions and their use in the preparation of drugs for the prevention and/or treatment of tumours comprising: ascorbic acid or derivatives thereof, and a second therapeutic agent Porcupine inhibitor.

[6]  Group 5: claims 1, 10-19, 20, 21, 23 and 31-41: pharmaceutical compositions comprising: ascorbic acid or a derivative thereof, and a second therapeutic agent, Niclosamide, the use of the combination of ascorbic acid or a derivative thereof with the second therapeutic agent in the preparation of drugs for the prevention and/or treatment of tumours, the second therapeutic agent being a Dvl-2 inhibitor.

[7]  Group 6: claims 1, 8, 10-19, 20, 21, 24 and 31-41: pharmaceutical compositions and their use in the preparation of drugs for the prevention and/or treatment of tumors comprising: ascorbic acid or derivatives thereof, and a second therapeutic agent CK1a inhibitor.

[8]  Group 7: Claims 1, 9-19, 20-21, 25 and 31-41: Pharmaceutical compositions and their use in the preparation of drugs for the prevention and/or treatment of tumors comprising: ascorbic acid or derivatives thereof, and a second therapeutic agent Frizzled receptor inhibitor.

Form PCT/ISA/210 (continuation of first sheet) (January 2015)

## INTERNATIONAL SEARCH REPORT

| International application No. |
| --- |
| **PCT/CN2021/084008** |

**Box No. III     Observations where unity of invention is lacking (Continuation of item 3 of first sheet)**

1. ☐  As all required additional search fees were timely paid by the applicant, this international search report covers all searchable claims.

2. ☐  As all searchable claims could be searched without effort justifying additional fees, this Authority did not invite payment of additional fees.

3. ☐  As only some of the required additional search fees were timely paid by the applicant, this international search report covers only those claims for which fees were paid, specifically claims Nos.:

4. ☑  No required additional search fees were timely paid by the applicant. Consequently, this international search report is restricted to the invention first mentioned in the claims; it is covered by claims Nos.: **Claims 1, 3, 4, 10-19, 20, 27, 28 and 31-41**

**Remark on Protest**     ☐  The additional search fees were accompanied by the applicant's protest and, where applicable, the payment of a protest fee.

☐  The additional search fees were accompanied by the applicant's protest but the applicable protest fee was not paid within the time limit specified in the invitation.

☐  No protest accompanied the payment of additional search fees.

Form PCT/ISA/210 (continuation of first sheet) (January 2015)

**INTERNATIONAL SEARCH REPORT**
Information on patent family members

International application No.

**PCT/CN2021/084008**

| Patent document cited in search report | | | Publication date (day/month/year) | Patent family member(s) | | | Publication date (day/month/year) |
|---|---|---|---|---|---|---|---|
| CN | 104136027 | A | 05 November 2014 | ES | 2660782 | T3 | 26 March 2018 |
| | | | | IL | 233285 | D0 | 31 August 2014 |
| | | | | AU | 2012356033 | B2 | 03 August 2017 |
| | | | | JP | 2020125314 | A | 20 August 2020 |
| | | | | WO | 2013093885 | A1 | 27 June 2013 |
| | | | | SG | 10201605984R | A | 29 September 2016 |
| | | | | BR | 112014015105 | A8 | 27 June 2017 |
| | | | | RU | 2014127753 | A | 10 February 2016 |
| | | | | JP | 6373760 | B2 | 15 August 2018 |
| | | | | US | 2014329867 | A1 | 06 November 2014 |
| | | | | US | 2018224429 | A1 | 09 August 2018 |
| | | | | NZ | 627559 | A | 24 June 2016 |
| | | | | NO | 2793884 | T3 | 28 April 2018 |
| | | | | CN | 107434779 | A | 05 December 2017 |
| | | | | US | 9296682 | B2 | 29 March 2016 |
| | | | | JP | 2015506345 | A | 02 March 2015 |
| | | | | EP | 3332783 | A1 | 13 June 2018 |
| | | | | RU | 2631611 | C2 | 25 September 2017 |
| | | | | SI | 2793884 | T1 | 30 March 2018 |
| | | | | RS | 56907 | B1 | 31 May 2018 |
| | | | | EP | 2606884 | A1 | 26 June 2013 |
| | | | | US | 2016266095 | A1 | 15 September 2016 |
| | | | | MX | 355632 | B | 25 April 2018 |
| | | | | HK | 1203396 | A1 | 30 October 2015 |
| | | | | MX | 2014007544 | A | 10 November 2014 |
| | | | | EP | 2793884 | A1 | 29 October 2014 |
| | | | | JP | 2018172433 | A | 08 November 2018 |
| | | | | ZA | 201404965 | B | 26 April 2017 |
| | | | | CY | 1119964 | T1 | 12 December 2018 |
| | | | | HR | P20180348 | T1 | 06 April 2018 |
| | | | | CA | 2859740 | A1 | 27 June 2013 |
| | | | | HU | E036583 | T2 | 30 July 2018 |
| | | | | LT | 2793884 | T | 26 February 2018 |
| | | | | PL | 2793884 | T3 | 30 May 2018 |
| | | | | BR | 112014015105 | A2 | 13 June 2017 |
| | | | | AU | 2012356033 | A1 | 07 August 2014 |
| | | | | CN | 104136027 | B | 24 May 2017 |
| | | | | TR | 201802117 | T4 | 21 March 2018 |
| | | | | SG | 11201403418V | A | 30 July 2014 |
| | | | | PT | 2793884 | T | 26 February 2018 |
| | | | | US | 2019219566 | A1 | 18 July 2019 |
| | | | | US | 10274481 | B2 | 30 April 2019 |
| | | | | US | 10054581 | B1 | 21 August 2018 |
| | | | | EP | 2793884 | B1 | 29 November 2017 |
| | | | | DK | 2793884 | T3 | 05 March 2018 |
| WO | 2011060051 | A1 | 19 May 2011 | US | 2011178046 | A1 | 21 July 2011 |
| | | | | US | 2014187495 | A1 | 03 July 2014 |
| | | | | US | 8637493 | B2 | 28 January 2014 |
| CN | 105658787 | A | 08 June 2016 | WO | 2015055987 | A1 | 23 April 2015 |
| | | | | US | 2016257930 | A1 | 08 September 2016 |

Form PCT/ISA/210 (patent family annex) (January 2015)

INTERNATIONAL SEARCH REPORT
Information on patent family members

International application No.

**PCT/CN2021/084008**

| Patent document cited in search report | | | Publication date (day/month/year) | Patent family member(s) | | | Publication date (day/month/year) |
|---|---|---|---|---|---|---|---|
| | | | | GB | 201318126 | D0 | 27 November 2013 |
| | | | | JP | 2016535586 | A | 17 November 2016 |
| | | | | EP | 3058064 | A1 | 24 August 2016 |
| CN | 107075471 | A | 18 August 2017 | SG | 11201701844Y | A | 27 April 2017 |
| | | | | EP | 3204489 | A4 | 05 September 2018 |
| | | | | EP | 3204489 | A1 | 16 August 2017 |
| | | | | JP | 2017534269 | A | 24 November 2017 |
| | | | | US | 2017304369 | A1 | 26 October 2017 |
| | | | | WO | 2016056999 | A1 | 14 April 2016 |
| CN | 110923191 | A | 27 March 2020 | None | | | |

Form PCT/ISA/210 (patent family annex) (January 2015)

## REFERENCES CITED IN THE DESCRIPTION

**Patent documents cited in the description**

- US 5137723 A
- US 5078989 A
- US 20050113312 A

**Non-patent literature cited in the description**

- **NIHRO et al.** *Chem. Pharm. Bull.,* 1991, vol. 39, 1731-1735
- **YAMAMOTO.** *J.Med.Chem.,* 2002, vol. 45 (2), 462-468